(19) Europäisches Patentamt
European Patent Office
Office européen des brevets

(11) EP 0 707 575 B1

(12) # FASCICULE DE BREVET EUROPEEN

(45) Date de publication et mention de la délivrance du brevet:
**24.10.2001 Bulletin 2001/43**

(21) Numéro de dépôt: **95911475.2**

(22) Date de dépôt: **27.03.1995**

(51) Int Cl.[7]: **C07D 307/83**, C07D 307/92, A61K 7/32, A61L 9/01

(86) Numéro de dépôt international:
**PCT/IB95/00206**

(87) Numéro de publication internationale:
**WO 95/30667 (16.11.1995 Gazette 1995/49)**

(54) **UTILISATION DE DIHYDROBENZOFURANONES A TITRE D'INGREDIENTS PARFUMANTS**
VERWENDUNG VON DIHYDROBENZOFURANONEN ALS PARFÜMINHALTSTOFFE
USE OF DIHYDROBENZOFURANONES AS SCENTING INGREDIENTS

(84) Etats contractants désignés:
**CH DE FR GB LI NL**

(30) Priorité: **09.05.1994 CH 144194**

(43) Date de publication de la demande:
**24.04.1996 Bulletin 1996/17**

(73) Titulaire: **FIRMENICH SA**
**1211 Genève 8 (CH)**

(72) Inventeurs:
- **VIAL, Christian**
**CH-1202 Genève (CH)**
- **BLANC, Pierre, Alain**
**CH-1263 Crassier (CH)**

(74) Mandataire:
**Salvaterra-Garcia, Maria de Lurdes et al**
**Firmenich SA**
**Département des Brevets**
**Case Postale 239**
**1211 Genève 8 (CH)**

(56) Documents cités:
**EP-A- 0 041 122** **WO-A-94/12143**
**US-A- 4 252 817**

- **CHEMICAL AND PHARMACEUTICAL BULLETIN, vol. 28,no. 1, 1980 TOKYO JP, pages 177-180, K. TAKAHASHI ET AL. 'Usnic Acid. XV. Alkaline Degradation of Usnic Acid'**
- **JOURNAL OF THE CHEMICAL SOCIETY, 1955 LETCHWORTH GB, pages 2166-2170, F.M. DEAN ET AL. 'Usnic Acid. Part XI. A Synthesis of 7-Acetyl-4:6-dihydroxy-3:5-dimethylcoumara n-2-one'**
- **CHEMICAL ABSTRACTS, vol. 104, no. 7, 17 Février 1986 Columbus, Ohio, US; abstract no. 50742e, O. PICCOLO ET AL 'A simple route to benzofuran-2(3H)-ones' page 508; colonne 1; cité dans la demande & J. CHEM. RES., SYNOP., no. 8, 1985 pages 258-259, ...**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Printed by Jouve, 75001 PARIS (FR)

EP 0 707 575 B1

## Description

### Domaine technique

La présente invention a trait au domaine de la parfumerie. Plus particulièrement, elle concerne l'utilisation, à titre d'ingrédient parfumant, d'un composé de formule

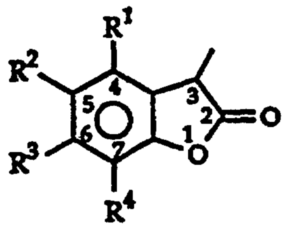

(I)

dans laquelle

a. les symboles $R^1$ à $R^4$ sont identiques ou différents et représentent chacun un atome d'hydrogène, un radical alkyle saturé ou insaturé, linéaire ou ramifié, ayant de 1 à 5 atomes de carbone, un radical alkoxy linéaire ou ramifié, ayant de 1 à 4 atomes de carbone, un radical cycloaliphatique ayant 5 ou 6 atomes de carbone ou un radical aromatique, ce radical cyclique pouvant être substitué par un ou plusieurs radicaux alkyles inférieurs ; ou dans laquelle.

b. deux symboles adjacents parmi les symboles $R^1$ à $R^4$ sont pris ensemble et représentent un cycle, saturé ou insaturé, ayant 5 ou 6 atomes de carbone, ce cycle pouvant être substitué par un ou plusieurs radicaux alkyles inférieurs, et les deux autres symboles représentent l'hydrogène.

Par radical alkyle inférieur on entend ici un radical de $C_1$ à $C_4$, linéaire ou ramifié, et plus particulièrement un radical méthyle ou éthyle.

### Technique antérieure

Les composés de formule (I) sont des analogues de la 3H-benzo[b]-furan-2-one, un composé dont la structure est bien connue.

Certains de ces composés et d'autres analogues de structure proche sont décrits dans l'art antérieur. On peut citer à ce propos la demande de brevet US-A-4 252 817 qui décrit des dérivés substitués de la 2,3-dihydrobenzofuran-2-one, parmi lesquels par exemple la 3-méthyl-5-méthoxy-6-cyclohexyle-2,3-dihydrobenzo-2-furanone. On peut également citer un autre composé obéissant à la formule (I), à savoir la 4,6-diméthoxy-3,7-diméthyl-3H-benzo[b]-furan-2-one qui est décrit par F.M. Dean & al. dans Journal of Chemical Society, 1955, pages 2166-2170. D'autres analogues de la 3H-benzo[b]-furan-2-one sont encore cités dans Chemical & Pharmaceutical Bulletin, vol. 28, no. 1, 1980, pages 177-180 ou encore dans Chemical Abstracts, vol. 104, no. 7, 1986, page 508. Tous ces documents décrivent les structures de ces composés et parfois leur préparation, mais il n'existe aucune description de leurs éventuelles propriétés organoleptiques.

Par ailleurs, on sait que plusieurs benzofuran-2(3H)-ones possèdent des propriétés thérapeutiques, notamment en tant qu'agents anti-inflammatoires (voir, par exemple, O. Piccolo et al., J. Chem. Res. 1985, 285).

Ainsi, nous n'avons pu déceler dans l'art antérieur aucune description, ni même suggestion, ayant trait à l'éventuelle utilité de tels composés dans la parfumerie. A l'évidence, les propriétés olfactives de ces composés sont passées totalement inaperçues jusqu'à aujourd'hui.

### Exposé de l'invention

Or, nous avons maintenant découvert que les composés de formule (I) possèdent des propriétés odorantes très utiles et qu'ils peuvent de ce fait servir à préparer des compositions parfumantes et articles parfumés de nature très variée.

Les notes olfactives des composés (I) couvrent en effet un spectre de nuances odorantes très large. Alors que l'on trouve certains composés (I) dont la note dominante est de type lactonique, coumariné, foin, rappellant l'odeur de la flouve et le caractère fèves de tonka, il en est d'autres qui développent des notes plutôt lactoniques où le caractère

fruité, abricot est dominant, ou encore des composés dont l'odeur est de type musqué, rappelant celle caractéristique des muscs nitrés (musc cétone, musc ambrette, etc), ou phénolique, mousse cristal. Certains, d'ailleurs, possèdent des odeurs où plusieurs de ces caractères se trouvent mélangés.

Il est donc apparent que l'utilisation des composés (I) en parfuaterie permet d'obtenir des effets parfumants très divers et que ces composés peuvent servir à impartir ou renforcer les notes coumarinées, lactoniques-fruitées, ou encore musquées, des compositions dans lesquelles ils sont incorporés.

Les propriétés odorantes de ces composés sont d'autant plus surprenantes qu'elles ne se trouvent pas représentées, de façon similaire, dans d'autres familles de composés dont la structure est pourtant très proche de celle des composés (I). C'est ainsi, par exemple, que nous avons observé que des homologues de ces composés dépourvus de substituant en position 3 du cycle lactonique, ou encore possédant deux substituants à la même position, se révélaient être de médiocres ingrédients parfumants par rapport au composé (I) correspondant, ou étaient même complètement dénués de caractère olfactif. Nous avons ainsi constaté, à plusieurs reprises, que la présence d'un substituant unique à la position 3 du squelette moléculaire de base, c'est-à-dire de la structure de type 3H-benzo[b]furan-2-one, semblait être une condition nécessaire pour la possession de propriétés odorantes utiles.

On peut citer à cet effet, le cas de la 3,6-diméthyl-3H-benzo[b]furan-2-one, un composé dont l'utilisation en parfumerie est préférée selon l'invention. Cette lactone développe une note odorante coumarinée très puissante, avec un caractère doux, foin, rappelant l'odeur de la flouve, et aussi une note de type tonka et vanillée. Or, cette combinaison de caractères olfactifs, et notamment la puissance du caractère coumariné-tonka qui rend ce composé particulièrement précieux pour un emploi alternatif à celui de la coumarine, ne se trouve pas représentée dans l'odeur faiblement coumarinée et phénolique de la 3,3,6-triméthyl-3H-benzo[b]furan-2-one et encore moins chez la 3-éthyl-3,6-diméthyl-3H-benzo[b]furan-2-one, qui possède une odeur désagréable rappelant les hydrocarbures. Par ailleurs, il s'est trouvé que cette odeur d'hydrocarbure caractérise aussi la note de la 3,6-diméthyl-3-propyl-3H-benzo[b]furan-2-one, alors que la 3-butyl-3,6-diméthyl-3H-benzo[b]furan-2-one possède une très faible odeur à caractère vaguement lactonique.

Le même type de comportement a été constaté avec la 6-isopropyl-3-méthyl-3H-benzo[b]furan-2-one et ses homologues supérieurs doublement substitués en position 3. Cette lactone nouvelle est ainsi un composé préféré de l'invention, son odeur étant représentative des notes dominantes de type lactonique, fruité, abricot dtées plus haut, accompagnées dans ce cas d'un léger sous-caractère phénolique. Il s'agit d'une odeur très tenace, qui rappelle celle de la décalactone et de la Veloutone (2,2,5-triméthyl-5-pentyl-1-cyclopentanone ; origine : Firmenich SA, Genève, Suisse). Or, c'est avec étonnement que les experts parfumeurs ont découvert que cette richesse olfactive avait complètement disparu chez la 6-isopropyl-3,3-diméthyl-3H-benzo[b]furan-2-one, dénuée de caractère olfactif, ou encore chez les 3-éthyl-6-isopropyl-3-méthyl-3H-benzo[b]furan-2-one, 6-isopropyl-3-méthyl-3-propylbenzo[b]furan-2-one et 3-butyl-6-isopropyl-3-méthyl-3H-benzo[b]furan-2-one, qui possèdent toutes des odeurs très faibles, à caractère très vaguement musqué ou lactonique.

Cette supériorité olfactive surprenante des composés (I) vis-à-vis de leurs homologues était aussi très évidente dans le cas de la 5,7-diisopropyl-3-méthyl-3H-benzo[b]furan-2-one et de la 5,7-di-tert-butyl-3-méthyl-3H-benzo [b]furan-2-one. Ces deux composés nouveaux, qui constituent des objets préférés de l'invention, possèdent des odeurs typiques de la troisième famille olfactive que l'on a découvert parmi les composés de l'invention, à savoir les odeurs à caractère musqué. Il s'agit d'odeurs d'autant plus inattendues qu'elles sont de type musc-nitré, malgré l'absence de groupes nitrés dans la molécule. En particulier, la lactone di-tert-butylée susmentionnée possède une odeur musquée très puissante, qui rappelle celle des muscs xylol et Baur. Un autre aspect de sa note musquée rappelle aussi l'odeur du musc cétone. Ce composé est en fait un ingrédient parfumant de choix et sa valeur olfactive s'est révélée encore plus riche lors de son utilisation en mélange particulier avec la 3-méthyl-cyclopentadéc-5-én-1-one (voir US 5,354,735) ou encore avec d'autres ingrédients musqués qui ne possèdent pas les notes caractéristiques desdits "muscs-nitrés", comme par exemple la Tonalid® (7-acétyl-1,1,3,4,4,6-hexaméthyltétraline; origine: PFW, Hollande).

Or, dans les deux cas, aussi bien leurs homologues inférieurs non méthylés en position 3, à savoir les 5,7-diisopropyl-3H-benzo[b]furan-2-one et 5,7-di-tert-butyl-3H-benzo[b]furan-2-one, que leurs homologues di-méthylés, ou 5,7-diisopropyl-3,3-diméthyl-3H-benzo[b]furan-2-one et 5,7-di-tert-butyl-3,3-diméthyl-3H-benzo[b]furan-2-one, ont des odeurs beaucoup trop faibles et dénuées de caractère olfactif bien défini.

De nombreux autres exemples du comportement olfactif surprenant des composés (I) ont été constatés. D'autre part, en plus de la remarquable gamme de nuances olfactives observée, certains types de lactones parmi les composés (I) semblaient éveiller une préférence particulière de la part des parfumeurs.

On peut citer, dans ce contexte, les composés de formule

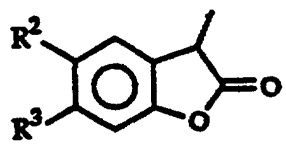

(Ia)

dans laquelle les symboles $R^2$ et $R^3$ sont identiques ou différents et représentent chacun un atome d'hydrogène ou un radical alkyle ou alkoxy de $C_1$ à $C_4$, linéaire ou ramifié, un radical cycloaliphatique ayant 5 ou 6 atomes de carbone ou un radical aromatique, ce radical cyclique pouvant être substitué par un ou plusieurs radicaux alkyles inférieurs.

On a déjà mentionné des exemples d'ingrédients de choix obéissant à la formule (Ia) et d'autres sont indiqués au Tableau I plus loin. On citera encore, à titre préférentiel, la 3-méthyl-6-phényl-3H-benzo[b]furan-2-one dont l'odeur lactonique, fruitée, abricot, légèrement grasse, rappelant celle des δ-lactones, de la γ-undécalactone et de la Veloutone, et notamment son extraordinaire ténacité, en font un ingrédient particulièrement prisé. En effet, la ténacité hors du commun de l'odeur de ce composé, dont l'intensité sur mouillette est restée inchangée pendant plus d'une année, dépasse de loin celle de n'importe quelle lactone connue à ce jour et rend ce composé très avantageux pour les applications typiques des lactones connues citées.

D'autres exemples intéressants parmi les composés (I), et dont on a déjà cité des exemples particulièrement préfères, sont les lactones de formule

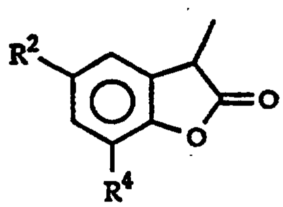

(Ib)

dans laquelle $R^2$ et $R^4$ sont identiques ou différents et représentent chacun un radical alkyle ou alkoxy de $C_1$ à $C_4$, linéaire ou ramifié, un radical cycloaliphatique ayant 5 ou 6 atomes de carbone ou un radical aromatique, ce radical cyclique pouvant être substitué par un ou plusieurs radicaux alkyles inférieurs.

Finalement, le tableau I ci-après cite encore d'autres composés préférés et leurs caractères olfactifs.

Tableau I

| N° | Composé | Propriétés odorantes |
|---|---|---|
| 1 | 3-méthyl-5-propyl-3H-benzo[b] furan-2-one | lactonique, foin, flouve, son |
| 2 | 5-tert-butyl-3-méthyl-3H-benzo[b] furan-2-one | lactonique, poudré, boisé, mousse, particulièrement tenace, rappelant l'acétate de p-tert-butyl-cyclohexyle |
| 3 | 3-méthyl-5-(1-méthylpropyl)-3H-benzo[b]furan-2-one | lactonique, fruité pêche et framboise, avec une sous-note vanillée, très puissant |
| 4 | 6-méthoxy-3-méthyl-3H-benzo[b] furan-2-one | coumariné, foin, flouve, puissant ; en fond légèrement phénolique |
| 5 | 3,6,7-triméthyl-3H-benzo[b]furan-2-one | coumariné, lactonique, un peu pêche, léger côté phénolique, avec une note de fond puissante |
| 6 | 3,5,6-triméthyl-3H-benzo[b]furan-2-one | coumariné, lactonique, noix de coco |
| 7 | 3,4,6-triméthyl-3H-benzo[b]furan-2-one | coumariné, rappelant l'odeur d'amandes amères, benzoïque, agréable |
| 8 | 3,5,6,7-tétrahydro-3-méthyl-indéno[5,6-b]furan-2-one | lactonique, puissant, noix de coco, flouve ; en fond, coumariné, tonka |
| 9 | 3-méthyl-3H-benzo[b]furan-2-one | tonka, coumariné-sec, puissant, benzoïque, doux |

Tableau I (suite)

| N° | Composé | Propriétés odorantes |
|---|---|---|
| 10 | 6-étltyl-3-méthyl-3H-benzo[b] furan-2-one | lactonique, coumariné, légèrement phénolique, mousse cristal, rappelant l'odeur du lilas et du Florec ®1) |

1) 9(10)-éthylidène-3-oxatricyclo[6.2.1.0$^{2,7}$]undécan-4-one ; origine : Firmenich SA, Genève, Suisse

De par leurs qualités olfactives, les composés (I) sont des ingrédients parfumants qui se prêtent tout aussi bien pour des applications en parfumerie fine qu'en parfumerie fonctionnelle. Ils conviennent pour la préparation de bases et compositions parfumantes, parfums et eaux de toilette, ainsi que pour le parfumage d'articles divers tels des savons, des gels de bain ou douche, des shampoings et autres produits d'hygiène capillaire, des désodorisants corporels ou d'air ambiant et des préparations cosmétiques. On peut également les utiliser avantageusement pour le parfumage de détergents et d'adoucissants textiles et ils trouvent aussi un emploi heureux dans des produits d'entretien.

Dans ces applications, ils seront utilisés soit seuls, soit, comme il est plus courant dans l'art, en mélange avec d'autres ingrédients parfumants, des solvants ou des adjuvants usuels en parfumerie.

Les concentrations dans lesquelles ils peuvent être utilisés dépendent de l'effet olfactif recherché, ainsi que de la nature des coingrédients avec lesquels ils sont mélangés dans les compositions parfumantes et articles parfumés les contenant. Par ailleurs, étant donné la variété de nuances olfactives que l'on peut trouver parmi les composés (I) et le spectre d'intensités odorantes correspondantes, il est clair que ces concentrations dépendront également de la nature du composé (I) utilisé. Ces concentrations peuvent donc varier dans une gamme de valeurs très large, dont on peut citer, purement à titre d'exemple, des concentrations de l'ordre de 1 à 10%, voire même 20% ou plus, en poids, par rapport au poids de la composition dans laquelle ils sont incorporés, ceci lors de leur usage dans la préparation de bases et compositions parfumantes. Des valeurs bien inférieures à celles-ci seront généralement utilisées lors de l'emploi de ces composés dans le parfumage des divers articles de consommation cités précédemment.

Les compositions parfumantes et les articles parfumés contenant les composés (I) et notamment les composés préférés cités auparavant, font aussi l'objet de l'invention.

Malgré le fait que l'on connait plusieurs composés obéissant à la formule (I), souvent cités dans le cadre d'études se rapportant à la synthèse organique ou à des activités dans le domaine pharmacologique, il est de ces composés qui sont des entités chimiques nouvelles, dont on ne pouvait anticiper de l'art antérieur qu'ils posséderaient des propriétés olfactives utiles.

Un autre objet de l'invention est ainsi un composé de formule

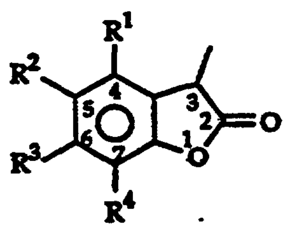

(I)

telle que définie à la revendication 1, alinéa a., étant entendu que les combinaisons suivantes sont exdues :

a. $R^1 = R^2 = R^3 = R^4 = H$;
b. $R^1 = R^2 = R^4 = H$ et $R^3$ = méthyle ou méthoxy ou phényle ou 2-méthylpropyle ou cydopentyle ou cyclohexyle ;
c. $R^1 = R^3 = R^4 = H$ et $R^2$ = méthyle ou éthyle ou phényle ;
d. $R^1 = R^4 = H$, $R^2 = CH_3$ et $R^3$ = cyclopentyle ou cyclohexyle;
e. $R^1 = R^4 = H$, $R^2$ = phényle et $R^3$ = méthoxy; et
f. $R^1 = R^3$ = méthoxy, $R^2 = CH_3$ et $R^4 = H$;

ou telle que définie à la revendication 1, alinéa b., mais en excluant la 1,5,8-triméthyl-1H-naphto[2,1-b]furan-2-one, la 3-méthyl-3H-naphto[2,3-b]furan-2-one et la 1-méthyl-1H-naphto[2,1-b]furan-2-one.

Par ailleurs, l'invention concerne en particulier les composés choisis dans le groupe constitué par

a. 6-éthyl-3-méthyl-3H-benzo[b]furan-2-one ;
b. 6-isopropyl-3-méthyl-3H-benzo[b]furan-2-one ;
c. 3-méthyl-5-propyl-3H-benzo[b]furan-2-one ;
d. 5-tert-butyl-3-méthyl-3H-benzo[b]furan-2-one ;
e. 3-méthyl-5-(1-méthylpropyl)-3H-benzo[b]furan-2-one ;
f. 3,4,6-triméthyl-3H-benzo[b]furan-2-one ;
g. 3,5,6-triméthyl-3H-benzo[b]furan-2-one ;
h. 3,6,7-triméthyl-3H-benzo[b]furan-2-one ;
i. 3,5,6,7-tétrahydro-3-méthyl-indéno[5,6-b]furan-2-one ;
j. 5,7-diisopropyl-3-méthyl-3H-benzo[b]furan-2-one; et
k. 5,7-di-tert-butyl-3-méthyl-3H-benzo[b]furan-2-one.

Les composés de formule (I) peuvent être préparés à partir de dérivés phénoliques disponibles sur le marché, ou qui peuvent être facilement préparés à partir de produits commerciaux. Les dérivés phénoliques de départ peuvent être transformés de façon analogue à la méthode décrite par O. Piccolo et al., réf. citée, représentée schématiquement comme suit:

Schéma I

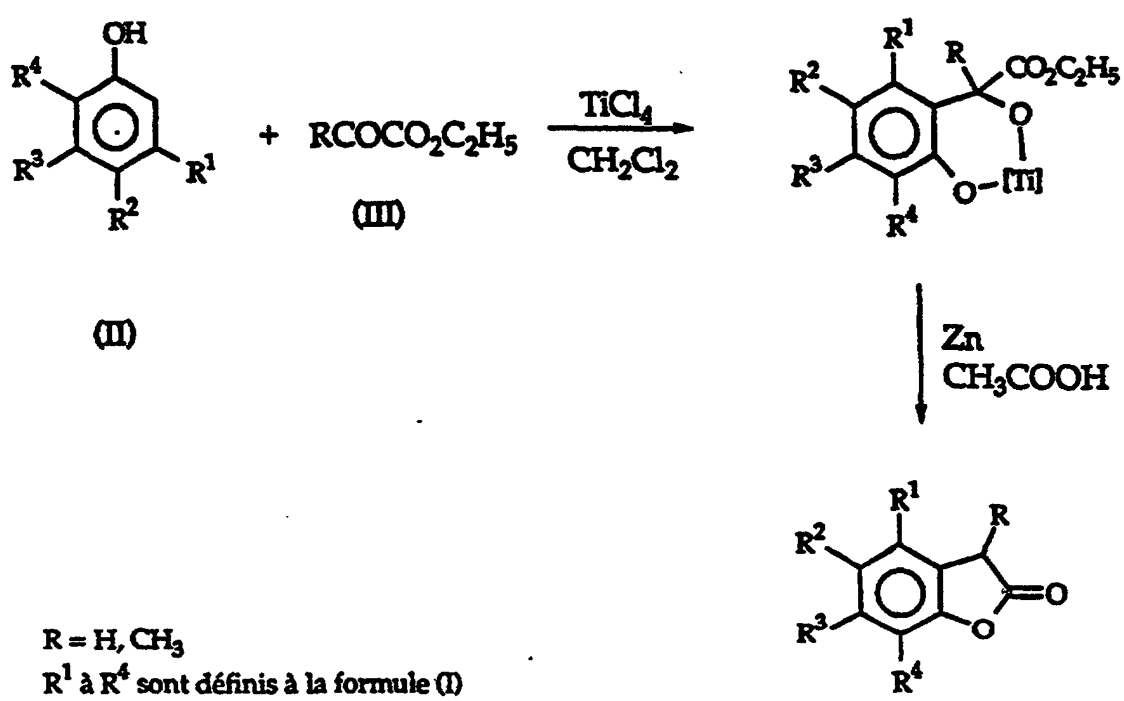


L'α-céto-ester de formule (III) dans lequel R = H, $CH_3$ est un produit commercial.

Alternativement, et en partant des mêmes dérivés phénoliques de formule (II), on peut préparer les composés (I) selon un procédé original, qui fait également l'objet de l'invention, caractérisé en ce qu'on fait réagir un dérivé phénolique de formule

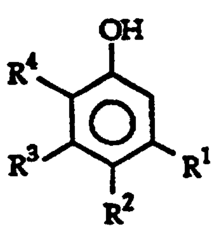

(II)

dans laquelle les symboles $R^1$ à $R^4$ sont définis comme à la formule (I), avec le méthylglyoxal, en présence d'un catalyseur acide et dans un solvant organique susceptible de former un mélange azéotropique avec l'eau.

Le procédé de l'invention repose sur une modification originale du procédé décrit par R. W. Layer dans J. Heterocycl. Chem. 12, 1067 (1975), représenté au schéma suivant :

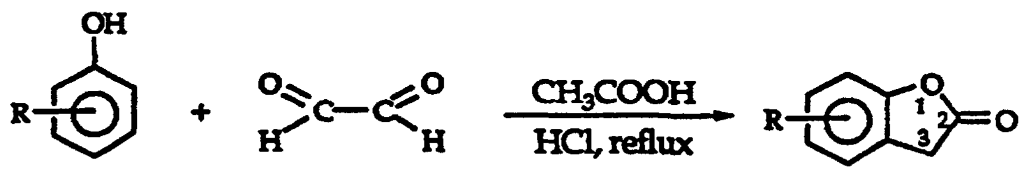


Nous avons constaté que, contrairement au procédé de l'invention, cette méthode connue ne permettait pas de préparer, de façon efficace, des composés dans lesquels le cycle lactonique est substitué en position 3. On observait, en effet, la formation d'une quantité prépondérante de produits à poids moléculaire élevé non intéressants.

Or, nous avons découvert avec surprise que lorsqu'on effectuait la réaction dans un solvant organique susceptible de former un mélange azéotropique avec l'eau, et permettant donc l'élimination de cette dernière, on palliait le problème constaté avec le procédé connu susmentionné.

A titre de catalyseur acide dans le procédé selon l'invention, on peut utiliser un acide organique ou minéral d'usage courant dans cette fonction, ou bien une résine acide. De préférence, on utilisera des acides sulfoniques, par exemple l'acide p-toluènesulfonique ou les résines sulfoniques telle que l'Amberlyst ® (origine : Rohm & Haas Co.) ou le Dowex® (origine : Dow Chem. Co).

Des solvants organiques pouvant être utilisés dans ce procédé appartiennent à la classe des hydrocarbures, notamment le toluène, le benzène, le cyclohexane ou encore le xylène, ou des solvants chlorés d'usage courant. De très bons rendements sont obtenus avec le toluène, par exemple.

La réaction peut être conduite à pression atmosphérique et à la température de reflux du solvant, ou bien sous vide à une température appropriée à la distillation azéotropique de l'eau.

Les dérivés phénoliques de formule (II), ainsi que le méthylglyoxal, utilisés comme produits de départ, peuvent être obtenus dans le commerce ou facilement préparés à partir de composés commerciaux, selon des méthodes classiques.

L'invention sera maintenant décrite plus en détail, à l'aide des exemples suivants, dans lesquels les températures sont indiquées en degrés centigrades et les abréviations ont le sens usuel dans l'art

## Manières de réaliser l'invention

### Exemple 1

Préparation des composés (I)

Méthode générale (I) (selon Citterio et al.)

On a suivi, de façon analogue, en utilisant les phénols de départ indiqués au tableau ci-après, la méthode décrite par O. Piccolo et al., réf. citée, page 259 et par A. Citterio et al., Synthesis, 1984, page 763. Les composés ont été purifiés à l'aide des techniques usuelles, a savoir chromatographie, distillation, etc ... Les homologues des composés (I) non méthylés en position 3 sont préparés de façon analogue (voir schéma I).

Méthode générale II (selon l'invention)

Dans un ballon tricol surmonté d'un séparateur d'eau de type Dean-Stark, d'un thermomètre et d'une ampoule d'introduction, placer 0,1 mole de phénol, 150 ml de toluène et 1,0 g d'acide p-toluènesulfonique. Chauffer à reflux (110°) et introduire lentement (en 1h30) 0,1 mole de méthylglyoxal (solutions aqueuses à 40%, Fluka) en maintenant le reflux pour éliminer l'eau. Chauffer encore 1h à reflux, refroidir à température ambiante, laver la phase organique avec une solution saturée de $NaHCO_3$ puis à l'eau jusqu'à neutralité, sécher sur $Na_2SO_4$, concentrer et purifier par chromatographie sur colonne ($SiO_2$) et par distillation ou cristallisation selon les cas.
Bien entendu, lorsque l'on remplace dans ce procédé le méthylglyoxal par le glyoxal, on obtient à nouveau les homologues inférieurs des composés de l'invention correspondants, c'est-à-dire les lactones non substituées en position 3 du cycle lactonique.

D'autre part, les homologues supérieurs des composés (I) qui sont di-substitués en position 3 sont préparés par alkylation, dans des conditions connues en soi, des composés de l'invention appropriés.
Plusieurs de ces composés homologues, choisis notamment parmi ceux cités plus haut, sont également décrits ci-après.

Le Tableau II suivant cite les composés préparés et leurs propriétés odorantes :

Tableau II

| N° | Produit de départ | Produit final | Propriétés odorantes du produit final |
|---|---|---|---|
| 1 | phénol | composé 9, Tableau I | voir Tableau I |
| 2 | 4-méthylphénol | 3,5-diméthyl-3H-benzo[b]furan-2-one | très mousse cristal, phénolique, légèrement coumariné |
| 3 | 3-méthylphénol | 3,6-diméthyl-3H-benzo[b]furan-2-one | voir plus haut |
| 4 | 2-méthylphénol | 3,7-diméthyl-3H-benzo[b]furan-2-one | sec, mousse cristal, algues |
| 5 | 3-éthylphénol | composé 10, Tableau I | voir Tableau I |
| 6 | 4-éthylphénol | 5-éthyl-3-méthyl-3H-benzo[b] furan-2-one | mousse cristal, phénolique |
| 7 | 4-propylphénol | composé 1, Tableau I | voir Tableau I |
| 8 | 3-isopropylphénol | 6-isopropyl-3-méthyl-3H-benzo [b]furan-2-one | voir plus haut |
| 9 | 4-isopropylphénol | 5-isopropyl-3-méthyl-3H-benzo [b]furan-2-one | phénolique, feuilles de thym, lactonique |
| 10 | 3-tert-butylphénol | 6-tert-butyl-3-méthyl-3H-benzo[b] furan-2-one | doux, vanillé |
| 11 | 4-tert-butylphénol | composé 2, Tableau I | voir Tableau I |
| 12 | 4-(1-méthylpropyl)-phénol | composé 3, tableau I | voir Tableau I |
| 13 | 3-méthoxyphénol | composé 4, Tableau I | voir Tableau I |
| 14 | 4-méthoxyphénol | 5-méthoxy-3-méthyl-3H-benzo[b] furan-2-one | phénolique, faiblement coumariné, vanillé |
| 15 | 2,3-diméthylphénol | composé 5, Tableau I | voir Tableau I |
| 16 | 2,4-diméthylphénol | 3,5,7-triméthyl-3H-benzo[b]furan-2-one | Florex® , coumariné, lactonique, flouve, rhubarbe-métallique, gras |
| 17 | 3,4-diméthylphénol | composé 6, Tableau I | voir Tableau I |
| 18 | 3,5-diméthylphénol | composé 7, Tableau I | voir Tableau I |
| 19 | 2,4,5-triméthylphénol | 3,4,6,7-tétraméthyl-3H-benzo[b] furan-2-one | phénolique, cirage |
| 20 | 3-phénylphénol | 3-méthyl-6-phényl-3H-benzo[b] furan-2-one | voir plus haut |
| 21 | 2-phénylphénol | 3-méthyl-7-phényl-3H-benzo[b] furan-2-one | phénolique, coumariné |
| 22 | 5,6,7,8-tétrahydro-1-naphtalénol | 6,7,8,9-tétrahydro-3-méthyl-3H-naphto [1,2-b]furan-2-one | vaguement humique; en fond, coumariné, flouve, phénolique |
| 23 | 5-indanol | composé 8, Tableau I | voir Tableau I |

Tableau II (suite)

| N° | Produit de départ | Produit final | Propriétés odorantes du produit final |
|---|---|---|---|
| 24 | 3,4,5,6-tétrahydro-3,3,4α,5β, 6,6-hexaméthyl-1-naphtalénol | 5,6,7,8-tétrahydro-3,5,5,6α,7β, 8,8-heptaméthyl-3H-naphto [2,3-b]furan-2-one | vaguement musqué, lactonique ; en fond, musqué |
| 25 | 2,4-di-tert-butylphénol | 5,7-di-tert-butyl-3-méthyl-3H-benzo[b]furan-2-one | voir plus haut |
| 26 | 2,4-di-tert-butylphénol | 5,7-di-tert-butyl-3H-benzo[b] furan-2-one | vaguement musqué |
| 27 | 5,7-di-tert-butyl-3-méthyl-3H-benzo[b]furan-2-one | 5,7-di-tert-butyl-3,3-diméthyl-3H-benzo[b]furan-2-one | faible, sans caractère olfactif |
| 28 | 2,4-diisopropylphénol (voir H. Jendralla et al., Synthesis 1990, 827) | 5,7-diisopropyl-3H-benzo[b] furan-2-one | sans caractère olfactif |
| 29 | 2,4-diisopropylphénol | 5,7-diisopropyl-3-méthyl-3H-benzo[b]furan-2-one | voir plus haut |
| 30 | 5,7-diisopropyl-3-méthyl-3H-benzo[b]furan-2-one | 5,7-diisopropyl-3,3-diméthyl-3H-benzo[b]furan-2-one | sans caractère olfactif |
| 31 | 2-tert-butyl-4-éthylphénol | 7-tert-butyl-5-éthyl-3-méthyl-3H-benzo[b]furan-2-one | lactonique, noix de coco, pêche, légèrement foin |
| 32 | 4-(1,1-diméthylpropyl)-phénol | 5-(1,1-diméthylpropyl)-3-méthyl-3H-benzo[b]furan-2-one | framboise, fraise, liqueur |
| 33 | 2-tert-butyl-phénol | 7-tert-butyl-3-méthyl-3H-benzo[b] furan-2-one | verte, pyrazines, pois |
| 34 | 3,6-diméthyl-3H-benzo[b]furan-2-one | 3,3,6-triméthyl-3H-benzo[b]furan-2-one | voir plus haut |
| 35 | 6-isopropyl-3-méthyl-3H-benzo [b]furait-2-one | 6-isopropyl-3,3-diméthyl-3H-benzo[b]furan-2-one | voir plus haut |
| 36 | 6-isopropyl-3-méthyl-3H-benzo [b]furan-2-one | 6-isopropyl-3-méthyl-3-propyl-3H-benzo[b]furan-2-one | voir plus haut |

Les caractères analytiques des composés cités dans ce Tableau sont décrits après, dans le même ordre :

| Composé 1 | |
|---|---|
| Pureté | 99,8% |
| SM | $M^+$148 (98); m/e: 133 (2), 120 (96), 103 (4), 91 (100), 77 (10), 65 (14), 51 (15), 39(12) |
| $^1$H-RMN(360 MHz, $CDCl_3$) | 1,57(d:J = 7,3H), 3,72(q:J = 7,1H), 7,06-7,36 (m, 4H) δ ppm |
| $^{13}$C-RMN (90 MHz, $CDCl_3$) | 1q: 15,9; 5d: 38,4, 110,7, 123,9, 124,2, 128,8; 3s: 128,8, 153,5, 177,9 δ ppm |

| Composé 2 | |
|---|---|
| Pureté | 99,7% |
| P.F. | 37,5-39° |
| SM | $M^+$162 (72); m/e: 147 (1), 134 (100), 119 (15), 105 (14), 91 (46), 77 (14), 65 (10), 51 (12), 39 (10) |
| $^1$H-RMN (360 MHz, $CDCl_3$) | 1,56 (d: J = 7,3H), 2,35 (s, 3H), 3,68 (q: J = 7,1H), 6,97 (d: J = 7,1H), 7,07 (m, 2H) δ ppm |
| $^{13}$C-RMN (90 MHz, $CDCl_3$) | 2q: 15,9, 21,1; 4d: 38,5, 110,3, 124,4, 129,1; 4s: 128,7, 133,8, 151,4, 178,3 δ ppm |

| Composé 3 | |
|---|---|
| Pureté | 99,8% |
| P.F. | 41,5 - 43° |
| SM | $M^+$162 (82); m/e: 147 (2), 134 (100), 119 (22), 105 (16), 91 (58), 77 (14), 65 (12), 51 (12), 39 (14) |
| $^1$H-RMN (360 MHz, $CDCl_3$) | 1,53 (d: J = 7, 3H), 2,37 (s, 3H), 3,67 (q: J = 7,1H), 6,90 (s, 1H), 6,95 (d: J = 7, 1H), 7,12 (d: J = 7,1H) δ ppm |
| $^{13}$C-RMN (90 MHz, $CDCl_3$) | 2q: 16,0, 21,6; 4d: 38,2, 111,3, 123,5, 124,8; 4s: 125,8, 139,2, 153,6, 178,3, δ ppm |

| Composé 4 | |
|---|---|
| Pureté | 99,9% |
| P.F. | 33,5- 35° |
| SM | $M^+$162 (70); m/e: 147(1), 134 (100), 119 (20), 105 (15), 91 (54), 77 (14), 65 (10), 51 (12), 39 (12) |
| $^1$H-RMN (360 MHz, $CDCl_3$) | 1,56 (d: J = 7,3H), 2,32 (s, 3H), 3,72 (q: J = 7,1H), 7,01 - 7,12 (m, 3H) δ ppm |
| $^{13}$C-RMN (90 MHz, $CDCl_3$) | 2q: 15,1, 15,9; 4d: 38,8, 121,1, 124,0, 130,2; 4s: 121,0, 128,4, 152,0, 178,2 δ ppm |

| Composé 5 | |
|---|---|
| Pureté | 98,7% |
| SM | $M^+$176 (45); m/e: 161 (2), 148 (66), 133 (100), 115 (8), 105 (22), 91 (17), 77 (20), 65 (12), 51 (14), 39 (20) |
| $^1$H-BMN (360 MHz, $CDCl_3$) | 1,24 (t: J = 7,3H), 1,55 (d: J = 7,3H), 2,67 (q: J = 7, 2H), 3,68 (q: J = 7,1H), 6,95 (s, 1H), 6,98 (d: J = 7, 1H), 7,15 (d: J = 7,1H) δ ppm |
| $^{13}$C-RMN (90 MHz, $CDCl_3$) | 2q: 15,6, 16,0; 1t: 29,1; 4d: 38,3, 110,1, 2x 123,6; 4s: 126,0, 145,7, 153,7, 178,4 δ ppm |

| Composé 6 | |
|---|---|
| Pureté | 99,1% |
| SM | $M^+$176 (31); m/e: 161 (2), 148 (30), 133 (100), 115 (4), 105 (8), 91 (11), 77(16), 65 (8), 51 (12), 39 (12) |
| $^1$H-RMN (360 MHz, $CDCl_3$) | 1,23(t:J=7,3H),1,57(d:J = 7,3H),2,64(q:J=7, 2H), 3,70 (q: J = 7,1H), 6,99 (d: J = 7,1H), 7,10 (m, 2H) δ ppm |
| $^{13}$C-RMN (90 MHz, $CDCl_3$) | 2q: 15,9, 16,0; 1t: 28,6; 4d: 38,5, 110,3, 123,3, 128,0; 4s: 128,8, 140,4, 151,5, 178,3 δ ppm |

| Composé 7 | |
|---|---|
| Pureté | 99,2% |
| SM | $M^+$190 (17); m/e: 161 (14), 146 (1), 133 (100), 115 (4), 103 (5), 91 (5), 77 (7), 65 (2), 51 (2), 39 (1) |
| $^1$H-RMN (360 MHz, $CDCl_3$) | 0,94 (t: J = 7,3H) 1,57 (d: J = 7, 3H), 1,63 (m, 2H), 2,58 (t: J = 7, 2H), 3,70 (q: J = 7, 1H), 6,99 (d: J = 7, 1H), 7,07 (m, 2H) δ ppm |
| $^{13}$C-RMN (90 MHz, $CDCl_3$) | 2q: 13,7, 15,9; 2t: 24,9, 37,7; 4d: 38,5, 110,2, 123,8, 128,7; 4s: 128,6, 138,8, 151,6, 178,3 δ ppm |

| Composé 8 | |
|---|---|
| Pureté | 99,4% |
| SM | $M^+$190 (44); m/e: 175 (12), 162 (33), 147 (100), 128 (6), 119 (14), 103 (8), 91 (32), 77 (14), 65 (10), 51 (6), 39 (8) |
| $^{1}$H-RMN(360 MHz, $CDCl_3$) | 1,25 (d: J = 7,6H), 1,55 (d: J = 7,3H), 2,93 (sept: J = 7, 1H), 3,69 (q: J = 7, 1H), 6,97 (s, 1H), 7,01 (d: J = 7,1H), 7,17(d: J = 7,1H) δ ppm |
| $^{13}$C-RMN (90 MHz, $CDCl_3$) | 3q: 15,9; 2x 24,0; 5d: 34,4, 38,3, 128,6, 122,3, 123,6; 4s: 126,1, 150,5, 153,7, 178,3 δ ppm |

| Composé 9 | |
|---|---|
| Pureté | 99,4% |
| SM | $M^+$ 190 (20); m/e: 175 (24), 162 (8), 147(100),128 (4), 115 (6), 103 (4), 91 (16), 77 (8), 65 (4), 51 (3), 39 (2) |
| $^{1}$H-RMN (360 MHz, $CDCl_3$) | 1,25 (d: J = 7, 6H), 1,57 (d: J = 7,3H), 2,91 (sept.: J = 7,1H), 3,70 (q: J = 7, 1H), 7,01 (d: J = 7,1H), 7,13 (m, 2H) δ ppm |
| $^{13}$C-RMN (90 MHz, $CDCl_3$) | 3q: 15,9; 2x 24,3; 5d: 34,0, 38,6, 110,3, 121,8, 126,6; 4s: 128,7, 145,1, 151,6, 178,3 δ ppm |

| Composé 10 | |
|---|---|
| Pureté | 99,3% |
| P.F. | 73 - 75° |
| SM | $M^+$204 (30); m/e: 189 (84), 176 (6), 161 (100), 146 (5), 133 (29), 115 (18), 105 (12), 91 (21), 77 (10), 65 (6), 51 (4), 41 (4) |
| $^{1}$H-RMN (360 MHz, $CDCl_3$) | 1,32 (s, 9H), 1,54 (d: J = 7,3H), 3,67 (q: J = 7,1H), 7,11 - 7,20 (m, 3H) δ ppm |
| $^{13}$C-RMN (90 MHz, $CDCl_3$) | 4q: 15,9, 3 x 31,3; 4d: 38,2, 107,9, 121,0, 123,3; 5s: 35,1, 125,7, 152,9, 153,6, 178,3 δ ppm |

| Composé 11 | |
|---|---|
| Pureté | 100% |
| P.F. | 71 - 72° |
| SM | $M^+$204 (28); m/e: 189 (88), 161 (100), 146 (3), 133 (14), 115 (8), 105 (5), 91 (10), 77 (6), 65 (4), 51 (4), 41 (5) |
| $^{1}$H-RMN (360 MHz, $CDCl_3$) | 1,33 (s, 9H), 1,58 (d : J = 7,3H), 3,70 (q: J = 7,1H), 7,01 (d: J = 7,1H), 7,27(s,1H), 7,31 (d : J = 7,1H) δ ppm |
| $^{13}$C-RMN (90 MHz, $CDCl_3$) | 4q: 15,9, 3x 31,6; 4d: 38,7, 110,0, 120,8, 125,6; 5s: 34,7, 128,3, 147,4, 151,3, 178,4 δ ppm |

| Composé 12 (mélange 1:1 de deux diastéréoisomères) | |
|---|---|
| Pureté | 98,1% |
| SM | $M^+$204 (18); m/e: 189 (2), 175 (79), 161 (5), 147 (100), 133 (8), 115 (8), 103 (4), 91 (14), 77 (6), 65 (3), 51 (2),39 (2) |
| $^{1}$H-RMN (360 MHz, $CDCl_3$) | 0,83 (t: J = 7,3H); 1,23 (d: J = 7, 3H), 1,57 (d: J = 7, 3H), 1,60 (m, 2H), 2,49 (hex.: J = 7, 1H), 3,70 (q: J = 7,1H), 6,96 - 7,13 (m, 3H) δ ppm |
| $^{13}$C-RMN (90 MHz, $CDCl_3$) | 3q: 12,2, 15,9, 22,0/22,1;1t: 31,3/31,4; 5d: 38,6, 41,5, 110,3, 122,3/122,4, 127,2/127,3; 4s: 128,7, 143,9, 151,6, 178,3 δ ppm |

| Composé 13 | |
|---|---|
| Pureté | 96,5% |
| P.F. | 33 - 35° |
| SM | $M^+$178 (64); m/e: 163 (12), 150 (100), 135 (20), 121 (17), 107 (22), 91 (22), 77 (26), 65 (12), 51 (10), 39 (8) |
| $^1$H-RMN (360 MHz, $CDCl_3$) | 1,53 (d: J = 7,3H), 3,67 (q: J =7,1H), 3,80 (s, 3H), 6,65 (s, 1H), 6,67 (d: J = 7,1H), 7,13 (d: J = 7,1H) δ ppm |
| $^{13}$C-RMN (90 MHz, $CDCl_3$) | 2q: 16,2, 55,6; 4d: 38,0, 97,5, 109,7, 124,3; 4s: 120,5, 154,4, 160,4, 178,4 δ ppm |

| Composé 14 | |
|---|---|
| Pureté | 99,5% |
| P.F. | 63 - 65° |
| SM | $M^+$178 (48); m/e: 163 (1), 150 (92), 135 (100), 121 (3), 107 (20), 91 (10), 77 (30), 63 (11), 53 (20), 39 (14) |
| $^1$H-RMN (360 MHz, $CDCl_3$) | 1,57 (d: J = 7,3H), 3,71 (q: J = 7,1H), 3,80 (s,3H), 6,81 (m,2H), 7,01 (d: J = 7,1H) δ ppm |
| $^{13}$C-RMN (90 MHz, $CDCl_3$) | 2q: 15,9, 55,9; 4d: 39,0, 110,2, 111,1, 113,5; 4s: 129,8, 147,3, 156,7, 178,3 δ ppm |

| Composé 15 | |
|---|---|
| Pureté | 99,8% |
| SM | $M^+$176 (47); m/e: 161 (2), 148 (100), 133 (53), 115 (16), 105 (62), 91 (26), 77 (22), 65 (10), 51 (10), 39 (8) |
| $^1$H-RMN (360 MHz, $CDCl_3$) | 1,54 (d: J = 7,3H), 2,22 (s, 3H), 2,29 (s, 3H), 3,71 (q: J = 7,1H), 6,93 (d: J = 7,1H), 6,97 (d: J = 7,1H) δ ppm |
| $^{13}$C-RMN (90 MHz, $CDCl_3$) | 3q: 11,8, 16,1, 19,6; 3d: 39,0, 120,5, 125,1; 5s: 119,7, 125,8, 137,9, 152,2, 178,5 δ ppm |

| Composé 16 | |
|---|---|
| Pureté | 99,3% |
| P.F. | 30 - 32° |
| SM | $M^+$176 (46); m/e: 161 (1), 148 (100), 133 (58), 115 (12), 105 (54), 91 (24), 77 (24), 65 (10), 51 (10), 39 (10) |
| $^1$H-RMN (360 MHz, $CDCl_3$) | 1,53 (d:J = 7, 3H), 2,27 (s, 3H), 2,31 (s, 3H), 3,67 (q: J = 7,1H), 6,87 (s, 1H), 6,90 (s, 1H) δ ppm |
| $^{13}$C-RMN (90 MHz, $CDCl_3$) | 3q: 14,9, 16,0, 21,0; 3d: 38,9, 121,6, 130,7; 5s: 120,5, 128,3, 133,5, 149,9, 178,5 δ ppm |

| Composé 17 | |
|---|---|
| Pureté | 100% |
| P.F. | 74 - 75° |
| SM | $M^+$176 (60); m/e: 161 (2), 148 (100), 133 (66), 115 (10), 105 (40), 91 (17), 77 (16), 65 (5),51 (4), 39 (4) |
| $^1$H-RMN (360 MHz, $CDCl_3$) | 1,51 (d: J = 7,3H), 2,23 (s, 3H), 2,25 (s, 3H), 3,62 (q: J = 7,1H), 6,84 (s, 1H), 7,00 (s, 1H) δ ppm |
| $^{13}$C-RMN (90 MHz, $CDCl_3$) | 3q: 16,0, 19,4, 20,1; 3d: 38,3, 111,6, 124,8; 5s: 126,0, 132,2, 137,3, 151,8, 178,5 δ ppm |

| Composé 18 | |
|---|---|
| Pureté | 99,8% |
| P.F. | 66-68° |
| SM | $M^+$176 (69); m/e: 161 (8), 148 (100), 133 (70), 115 (18), 105 (80), 91 (26), 77 (29), 65(11), 51 (12), 39 (13) |
| $^1$H-RMN (360 MHz, $CDCl_3$) | 1,56 (d: J = 7,3H), 2,29 (s, 3H), 2,32 (s, 3H), 3,64 (q: J = 7,1H), 6,73 (s, 1H), 6,75 (s, 1H) δ ppm |
| $^{13}$C-RMN (90 MHz, $CDCl_3$) | 3q: 15,6, 18,2, 21,5; 3d: 38,0, 108,8, 126,3; 5s: 124,0, 134,5, 138,9, 153,6, 178,5 δ ppm |

| Composé 19 | |
|---|---|
| Pureté | 100% |
| P.F. | 49 - 51° |
| SM | $M^+$190 (54); m/e: 175(5), 162(100), 147(78), 129 (9), 119(56), 105(10), 91(32), 77 (17), 65(10), 51 (7), 39 (8) |
| $^1$H-RMN (360 MHz, $CDCl_3$) | 1,57 (d: J = 7, 3H), 2,17 (s, 3H), 2,24 (s, 3H), 2,26 (s, 3H), 3,67 (q: J = 7,1H), 6,73 (s, 1H) δ ppm |
| $^{13}$C-RMN (90 MHz, $CDCl_3$) | 4q: 11,6, 15,7, 17,9, 19,4; 2d: 38,8, 126,6; 6s: 116,8, 123,9, 131,2, 137,6, 152,0, 178,7 δ ppm |

| Composé 20 | |
|---|---|
| Pureté | 99,6% |
| P.F. | 102 - 104° |
| SM | $M^+$224 (62); m/e: 209 (2), 196 (100), 181 (10), 167 (44), 152 (36), 139 (8), 128 (6), 115 (12), 102 (6), 89 (9), 76 (8), 63 (5), 51 (4), 39 (2) |
| $^1$H-RMN (360 MHz, $CDCl_3$) | 1,58 (d: J = 7,3H), 3,73 (q: J = 7,1H), 7,23 - 7,58 (m, 8H) δ ppm |
| $^{13}$C-RMN (90 MHz, $CDCl_3$) | 1q: 15,9; 9d: 38,3, 109,4, 123,0, 124,1, 2x 127,1, 127,8, 2x 128,9; 5s: 127,6, 140,2, 142,5, 154,0, 177,9 δ ppm |

| Composé 21 | |
|---|---|
| Pureté | 99,3% |
| P.F. | 99-102° |
| SM | $M^+$224 (62); m/e: 196 (52), 181 (100), 165 (22), 152 (30), 139(6), 128(4), 115(12), 98(6), 89 (4), 76(6), 63(6), 51(4), 39 (4) |
| $^1$H-RMN(360 MHz,$CDCl_3$) | 1,63 (d: J = 7,3H), 3,79 (q: J = 7,1H), 7,23 (m, 2H), 7,37 (m, 1H), 7,46 (m, 3H), 7,67 (m, 2H) δ ppm |
| $^{13}$C-RMN (90MHz, $CDCl_3$) | 1q: 16,0; 9d: 38,4, 122,7, 124,6, 127,9, 3x 128,6, 2x 128,9; 5s: 125,0, 129,5, 135,4, 150,5, 177,9 δ ppm |

| Composé 22 | |
|---|---|
| Pureté | 98,5% |
| P.F. | 63 - 65° |
| SM | $M^+$202 (60); m/e: 187 (1), 174 (100), 159 (66), 145 (28), 131 (33), 115(33), 103 (10), 91 (22), 77(16), 65 (8),51 (8), 39 (6) |
| $^1$H-RMN (360 MHz, $CDCl_3$) | 1,52 (d: J = 7,3H), 1,78 (m, 4H), 2,72 (m, 4H), 3,64 (q: J = 7,1H), 6,85 (d: J = 7,1H), 6,96 (d: J = 7,1H) δ ppm |

(suite)

| Composé 22 | |
|---|---|
| $^{13}$C-RMN (90 MHz, $CDCl_3$) | 1q: 16,0; 4t: 22,2, 2x 22,8, 29,4; 3d: 38,7, 120,3, 124,5; 5s: 120,8, 125,1, 138,5, 151,5, 178,6 δ ppm |

| Composé 23 | |
|---|---|
| Pureté | 97,6% |
| P.F. | 79 - 81° |
| SM | $M^+$188 (52); m/e: 173 (2), 160 (100), 145 (18), 133 (14), 115 (22), 103 (4), 91 (12), 77 (8), 63 (4), 51 (4), 39 (2) |
| $^1$H-RMN (360 MHz, $CDCl_3$) | 1,53 (d: J = 7, 3H), 2,10 (m, 2H), 2,89 (m, 4H), 3,67 (q: J = 7,1H), 6,95 (s, 1H), 7,09 (s, 1H) δ ppm |
| $^{13}$C-RMN (90 MHz, $CDCl_3$) | 1q: 16,2; 3t 25,8, 32,4, 33,2; 3d: 38,6,107,0,119,6; 5s: 126,6, 139,7, 144,9, 152,1, 178,7 δ ppm |

| Composé 24 (mélange 1:1 de deux diastéréoisomères) | |
|---|---|
| Pureté | 98% |
| P.F. | 127 - 130° |
| SM | $M^+$286 (29); m/e: 271 (91), 243 (4), 229 (48), 215 (100), 201 (30), 187 (12), 171 (6), 157(5), 141(8), 128(10), 115(8), 91 (7), 77 (6), 57 (23), 43 (12) |
| $^1$H-RMN (360 MHz, $CDCl_3$) | 0,98 (d: J = 6,6H), 1,10 (s, 6H), 1,30 (s, 6H), 1,57 (m, 5H), 3,68 (m, 1H), 7,07 (s, 1H), 7,23 (s, 1H) δ ppm |
| $^{13}$C-RMN (90 MHz, $CDCl_3$) | 7q: 13,9, 14,0, 15,9/16,0, 25,6/25,7, 25,8, 29,6/29,8, 29,8/29,9; 5d: 38,4/38,5,39,1, 39,2, 108,4, 122,4/122,5; 7s: 2x 38,0,126,2, 141,7, 146,9, 151,6, 178,6 δ ppm |

| Composé 25 | |
|---|---|
| Pureté | 99,6% |
| P.F. | 62 - 64° |
| SM | $M^+$260 (26); m/e: 245 (100), 232 (2), 217 (70), 201 (2), 189 (2), 175 (2), 159 (2), 145 (2), 128 (4),115 (6),105 (4),91 (6), 77 (4), 57(9), 41 (5) |
| $^1$H-RMN (360 MHz, $CDCl_3$) | 1,33 (s, 9H), 1,40 (s, 9H), 1,57 (d: J = 7,3H), 3,67 (q: J = 7,1H), 7,11 (s, 1H), 7,27 (s, 1H) δ ppm |
| $^{13}$C-RMN (90 MHz, $CDCl_3$) | 7q: 15,9, 3x 29,6, 3x 31,6; 3d: 38,2, 118,2, 122,8; 7s: 34,4, 34,8, 128,6, 133,3, 147,0, 149,2, 178,6 δ ppm |

| Composé 26 | |
|---|---|
| Pureté | 99,6% |
| P.F. | 89 - 92° |
| SM | $M^+$246 (22); m/e: 231 (100), 217 (1), 203 (52), 188 (2), 175 (3), 161 (6), 145 (7), 128 (6), 115 (10), 105 (8), 91 (12), 80 (9), 65 (4), 57 (6), 41 (8) |
| $^1$H-RMN (360 MHz, $CDCl_3$) | 1,32 (s, 9H), 1,40 (s, 9H), 3,68 (s, 2H), 7,15 (s, 1H), 7,26 (s, 1H) δ ppm |
| $^{13}$C-RMN (90 MHz, $CDCl_3$) | 6q: 3x 29,6, 3x 31,6; 1t: 32,9; 2d: 119,1, 122,7; 7s: 34,3, 34,7, 123,0, 133,3, 146,8, 150,5, 174,5 δ ppm |

| Composé 27 | |
|---|---|
| Pureté | 99,3% |
| P.F. | 114-115° |
| SM | $M^+$274 (26); m/e: 259 (90), 231 (100), 215(4), 201 (2), 189 (2), 173 (2), 159 (2), 141 (4), 128 (6), 115 (7), 108 (7), 94 (9), 80 (10), 65 (4), 57 (18), 41 (14) |
| $^1$H-RMN (360 MHz, $CDCl_3$) | 1,33 (s, 9H), 1,40 (s, 9H), 1,49 (s, 6H), 7,05 (s divisé, 1H), 7,26 (s divisé, 1H) δ ppm |
| $^{13}$C-KMN (90 MHz, $CDCl_3$) | 8q: 2x 25,5, 3x 29,7, 3x 31,7; 2d: 116,9, 122,6; 8s: 34,4,34,9,42,5,133,3,133,5,147,1,147,9,181,6 δ ppm |

| Composé 28 | |
|---|---|
| Pureté | 99,5% |
| P.F. | 29-31° |
| SM | $M^+$218 (41); m/e: 203 (100), 190 (4), 175 (89), 159 (3), 147 (27), 131 (6), 119 (14), 105 (13), 91 (19), 77 (13), 65 (7), 51 (6), 41 (10) |
| $^1$H-RMN (360 MHz, $CDCl_3$) | 1,23 (d: J = 7,6H), 1,28 (d: J = 7, 6H), 2,88 (sept.: J = 7,1H), 3,15 (sept.: J = 7, 1H), 3,69 (s, 2H), 6,98 (s, 1H) δ ppm |
| $^{13}$C-RMN (90 MHz, $CDCl_3$) | 4q: 2x 22,4, 2x 24,3; 1t: 33,4; 4d: 28,8, 34,1, 119,6, 124,4; 5s: 122,7, 131,4, 145,1, 150,4, 174,8 δ ppm |

| Composé 29 | |
|---|---|
| Pureté | 99,5% |
| SM | $M^+$232 (27); m/e: 217 (32), 204 (16), 189 (100), 175 (3), 161 (13), 147 (6), 128 (8), 115 (10), 105 (5), 91 (13), 77 (8), 65 (4), 53 (4), 41 (9) |
| $^1$H-RMN (360 MHz, $CDCl_3$) | 1,24 (d: J = 7, 6H), 1,29 (d divisé: J = 7,6H), 1,57 (d: J = 7,3H), 2,49 (sept.: J = 7, 1H), 3,15 (sept.: J = 7, 1H), 3,69 (q: J = 7, 1H), 6,93 (s, 1H), 7,01 (s, 1H) δ ppm |
| $^{13}$C-RMN (90 MHz, $CDCl_3$) | 5q: 15,9, 22,3, 22,5, 24,3, 24,4; 5d: 28,8, 34,1, 38,8, 118,8, 124,3; 5s: 128,4, 131,3, 145,1, 149,1, 178,7 δ ppm |

| Composé 30 | |
|---|---|
| Pureté | 99,3% |
| P.F. | 34 - 37° |
| SM | $M^+$246(29); m/e: 231 (21), 218 (24), 203 (100), 187 (2), 175 (8), 161 (3), 147 (3), 128 (7), 115 (8), 105 (5), 91 (8), 80 (6), 65 (4), 53 (3), 41 (9) |
| $^1$H-RMN (360 MHz, $CDCl_3$) | 1,25 (d: J = 7,6H), 1,29 (d: J = 7,6H), 1,49 (s, 6H), 2,90 (sept.: J = 7, 1H), 3,17 (sept.: J = 7, 1H). 6,88 (s divisé, 1H), 7,00 (s divisé, 1H) δ ppm |
| $^{13}$C-RMN (90 MHz, $CDCl_3$) | 6q: 2x 22,5, 2x 24,3, 2x 25,5; 4d: 28,7, 34,2, 117,7, 124,0; 6s: 43,2, 131,4, 133,3, 145,2, 147,8, 181,7 δ ppm |

| Composé 31 | |
|---|---|
| Pureté | 99,8% |
| P.F. | 57 - 58° |
| SM | $M^+$232 (24); m/e: 217 (30), 204 (6), 189 (100), 175 (10), 161 (16), 141 (4), 128 (8), 115 (8), 105 (5), 91 (10), 77·(6), 65 (4),53 (2), 41 (4) |
| $^1$H-RMN (360 MHz, $CDCl_3$) | 1,23 (t: J = 7,3H), 1,39 (s, 9H), 1,56 (d: J = 7,3H), 2,63 (q: J = 7,2H), 3,65(q: J = 7,1H), 6,93 (s, 1H), 7,05 (s, 1H) δ ppm |
| $^{13}$C-RMN (90 MHz, $CDCl_3$) | 5q: 15,9, 16,0, 3x29,6; 1t: 28,8; 3d: 38,0, 120,6, 125,4; 6s: 34,2, 129,2, 133,8, 140,0, 149,4, 178,5 δ ppm |

| Composé 32 | |
|---|---|
| Pureté | 96,8% |
| P.F. | 30 - 32° |
| SM | $M^+$218 (10); m/e: 203 (4), 189 (100), 175 (4), 161 (88), 145 (4), 133 (20), 115 (10), 105 (6), 91 (12), 77 (6), 65 (3), 55(2), 41(3) |
| $^1$H-RMN (360 MHz, $CDCl_3$) | 0,69 (t: J = 7,3H), 1,28 (s, 6H), 1,59 (d: J = 7,3H), 1,63 (q: J = 7,2H), 3,71(q: J = 7.1H), 7,01 (d: J = 7, 1H), 7,20 (s, 1H), 7,25 (d: J = 7,1H) δ ppm |
| $^{13}$C-RMN (90 MHz, $CDCl_3$) | 3q: 9,1, 16,0, 28,7; 1t: 37,0; 4d: 38,7, 109,9, 121,4, 126,3; 5s: 37,9, 128,3, 145,8, 151,3, 178,4 δ ppm |

| Composé 33 | |
|---|---|
| Pureté: | 98,3% |
| SM : | $M^+$204 (21); m/e: 189 (34), 176 (1), 161 (100), 147 (4), 133 (26), 115 (10), 105 (6), 91 (10), 77 (6), 65 (3),51(2),39(2) |
| $^1$H-RMN (360 MHz, $CDCl_3$): | 1,40(s, 9H), 1,57(d:J = 7,3H), 3,69 (q:J = 7, 1H), 7,07-7,27 (m, 3H) δ ppm |
| $^{13}$C-RMN (90 MHz, $CDCl_3$): | 4q: 15,9, 3x 29,6; 4d: 37,9, 121,4, 124,0, 125,9; 5s: 34,2, 129,2, 134,4, 151,4, 178,1 δ ppm |

| Composé 34 | |
|---|---|
| Pureté : | 99,9% |
| SM : | $M^+$176 (60); m/e: 161 (14), 148 (100), 133 (69), 115 (15), 105 (58), 91 (16), 77 (24), 63 (10), 51 (14), 39 (18) |
| $^1$H-RMN (360 MHz, $CDCl_3$): | 1,47 (s, 6H), 2,37 (s, 3H), 6,93 (s, 1H), 6,96 (d: J = 7, 1H), 7,09 (d:J = 7,1H) δ ppm |
| $^{13}$C-RMN (90 MHz, $CDCl_3$) : | 3q: 21,6, 2x 25,3; 3d: 111,4, 122,4, 124,9; 5s: 42,8, 130,7, 138,9, 152,3, 181,2 δ ppm |

| Composé 35 | |
|---|---|
| Pureté | 99,9% |
| P.F. | 77-78° |
| SM | $M^+$204 (39); m/e: 189 (16), 176 (64), 161 (100), 145 (4), 133 (15), 115 (13), 105 (9), 91 (16), 77 (12), 65 (8), 51 (8), 41 (14) |
| $^1$H-RMN (360 MHz, $CDCl_3$) | 1,26 (d: J = 7,6H), 1,48 (s, 6H), 2,93 (sept.: J = 7, 1H),6,99 (s, 1H), 7,02 (d: J = 7,1H), 7,12 (d: J = 7, 1H) δ ppm |
| $^{13}$C-RMN (90 MHz, $CDCl_3$) | 4q: 2x 24,0, 2x 25,3; 4d: 34,4, 108,8, 2x 122,4; 5s: 42,9, 130,9, 150,2, 152,4, 181,4 δ ppm |

| Composé 36 | |
|---|---|
| Pureté | 99,9% |
| SM | $M^+$232 (30); m/e: 217(3), 204 (5), 189 (100), 175 (14), 161 (36), 147 (26), 133 (10), 115 (13), 105 (9), 91 (16), 77 (8), 65 (5), 51 (4), 41 (14) |
| $^1$H-RMN (360 MHz, $CDCl_3$) | 0,83 (t: J = 7,3H), 0,93-1,22 (m, 2H), 1,27 (d: J = 7, 6H), 1,47 (s, 3H), 1,75 (m, 1H), 1,90 (m, 1H), 2,93 (sept.: J = 7, 1H), 6,97 (s divisé, 1H), 7,01 (d divisé, J = 7, 1H), 7,08 (d: J = 7, 1H) δ ppm |
| $^{13}$C-RMN(90 MHz, $CDCl_3$) | 4q: 14,0, 2x 24,0, 24,4; 2t: 18,1, 41,4; 4d: 34,3, 108,6, 122,3, 122,7; 5s: 47,4, 129,4, 150,1, 152,9, 181,0 δ ppm |

Exemple 2

Préparation d'une composition parfumante

On a préparé une composition parfumante de base, destinée à un parfum de type masculin, oriental, en mélangeant les ingrédients suivants :

| Ingrédients | Parties en poids |
|---|---|
| Acétate de linalyle | 210 |
| Ambrox® DL 1) à 10%* | 350 |
| Anthranilate de méthyle dist. | 25 |
| 4-(4-Hydroxyphényl)-2-butanone à 10%* | 15 |
| Essence de bergamote synth. | 135 |
| Essence de cannelle de Ceylan à 10%* | 70 |
| Eugénol à 10%* | 40 |
| Essence de citron | 110 |
| Héliotropine ord. | 120 |
| Hydroxycitronellal | 35 |
| Indol purif. à 10%* | 15 |
| Polywood® 2) | 190 |
| Dihydromyrcénol 3) | 180 |
| Linalol | 50 |
| Lyral® 4) | 400 |
| Essence de mandarine synth. | 30 |
| Iralia® 5) | 90 |
| Méthylnaphtylcétone cryst. | 20 |
| Jasmonate de méthyle | 300 |
| Essence de patchouli | 120 |
| Sandalore® 6) | 50 |
| Tonalid® 7) | 500 |
| Exaltolide® 8) | 200 |
| Vanilline | 95 |
| Vertofix coeur9) | 250 |
| TOTAL | 3600 |

* dans le dipropylèneglycol (DIPG)

1) tétraméthyl-perhydronaphtofurane, origine : Firmenich SA, Genève, Suisse

2) acétate de perhydro-5,5,8a-triméthyl-2-naphtyle ; origine: Firmenich SA, Genève, Suisse

3) origine : International Flavors and Fragrances Inc., U.S.A.

4) 4-(4-hydroxy-4-méthylpentyl)-3-cyclohexène-1-carboxaldéhyde ; origine : International Flavors and Fragrances Inc., U.S.A.

5) méthylionone ; origine : Firmenich SA, Genève, Suisse

6) 5-(2,2,3-triméthylcyclopent-3-ényl)-3-méthylpentan-2-ol; origine : Givaudan-Roure, Vernier, Suisse

7) 7-acétyl-1,1,3,4,4,6-hexaméthyltétraline; origine : PFW, Hollande

8) pentadécanolide ; origine : Firmenich SA, Genève, Suisse

9) origine : Internadonal Flavors and Fragrances Inc., U.S.A.

Lorsqu'on a ajouté à cette composition de base de type balsamique, floral, boisé, musqué, 400 parties en poids de 3,6-diméthyl-3H-benzo[b]furan-2-one, on a obtenu une composition nouvelle dont la note orientale, coumarinée-vanillée ressortait nettement renforcée, avec un caractère tonka bien marqué et une puissance nettement accrue. Cet effet olfactif coumariné, que l'on a observé également à des concentrations inférieures du composé de l'invention, de l'ordre de la moitié ou même un quart de la quantité citée, ressemble à celui que l'on aurait pu obtenir avec la coumarine, la note de type fèves de tonka étant cependant plus marquée.
Des effets semblables, mais toutefois nuancés, ont été obtenus lorsqu'on a ajouté la même quantité d'autres composés selon l'invention à odeur coumarinée. Par exemple, la 3-méthyl-3H-benzo[b]furan-2-one conférait une odeur du même type, mais plus sèche et plus douoe, alors que la 6-méthoxy-3-méthyl-3H-benzo[b]furan-2-one impartissait à la composition une odeur coumarinée, mais plus phénolique. L'adjonction de la 3,5,6,7-tétrahydro-3-méthyl-indéno[5,6-b]furan-2-one faisait également ressortir la note coumarinée-tonka dans l'odeur de la composition nouvelle, avec un caractère de tête de type noix de coco, que l'on retrouvait également lors de l'addition de la 3,5,6-triméthyl-3H-benzo [b]furan-2-one à la composition de base, alors que la 3,4,6-triméthyl-3H-benzo[b]furan-2-one conférait plutôt un caractère coumariné-amande amère à cette composition.

Exemple 3

Parfumage de linge

On a préparé des échantillons d'une base adoucissante parfumée en ajoutant à des échantillons distincts d'un adoucissant textile standard non parfumé les composés selon l'invention cités au Tableau suivant, dans les quantités indiquées :

| Ingrédients | Echantillon | | | | | | |
|---|---|---|---|---|---|---|---|
| | 1 | 2 | 3 | 4 | 5 | 6 | 7 |
| adoucissant non-parfumé | 99,9 | 99,9 | 99,9 | 99,9 | 99,9 | 99,9 | 99,9 |
| 3,6 diméthyl-3H-benzo[b]furan-2-one | 0,1 | - | - | - | - | | - |
| 6-isopropyl-3-méthyl-3H-benzo[b] furan-2-one | - | 0,1 | - | - | - | | - |
| 3-méthyl-6-phényl-3H-benzo[b] furan-2-one | - | - | 0,1 | - | - | | - |
| 6-éthyl-3-méthyl-3H-benzo[b]furan-2-one | - | - | - | 0,1 | - | | - |
| 6-méthoxy-3-méthyl-3H-benzo [b]furan-2-one | - | - | - | - | 0,1 | | - |
| 5,7-diisopropyl-3-méthyl-3H-benzo [b]furan-2-one | - | - | - | - | - | 0,1 | - |
| 5,7-di-tert-butyl-3-méthyl-3H-benzo [b]furan-2-one | | | | | | | 0,1 |

Dans sept machines à laver le linge, on a traité séparément sept lots de textiles standard, contenant des textiles en coton, en fibre acrylique et en nylon, avec respectivement les échantillons 1 à 7 préparés ci-dessus. Les sept lots de textiles ainsi traités ont ensuite été évalués à l'aveuglé par un panel d'experts parfumeurs, aussi bien à l'état humide qu'après leur séchage.

De l'avis des parfumeurs, on pouvait distinguer trois effets olfactifs distiricts. Ainsi, les textiles traités avec les échantillons 1, 4 et 5 développaient une odeur à caractère coumariné dominant, très puissante dans le cas du lot traité avec l'échantillon 1, avec un note foin, flouve et tonka bien marquée, alors que le lot traité avec l'échantillon 4 avait une odeur où le caractère lactonique et mousse cristal était plus marqué et qui était aussi plus phénolique.

Pour leur part, l'odeur des textiles traités avec les échantillons 2 et 3 n'était plus à caractère dominant coumariné, mais lactonique et fruité, très agréable évoquant l'odeur de la Veloutone (origine : Firmenich SA, Genève, Suisse) et des décalactones, et dont la note fruitée ressemblait à l'odeur d'abricot. Par ailleurs, l'odeur des textiles traités avec l'échantillon 3 s'est révélée particulièrement tenace, restant sur les tissus pendant longtemps.

D'autre part, les lots traités avec les échantillons 6 et 7 ont été jugés complètement différents des autres, leur odeur étant fortement musquée et pas du tout coumarinée ni lactonique-fruitée, avec un caractère de type musc nitré particulièrement apprécié.

Cet effet était d'autant plus puissant dans les textiles parfumés à l'aide de l'échantillon 7 et ceci aussi bien à la sortie de la machine que 24 h plus tard.

Selon les parfumeurs, ces variations dans les effets olfactifs observés avaient été trouvées tout à fait surprenantes lorsqu'ils ont constaté la nature des ingrédients parfumants utilisés et la similarité de leurs structures.

Exemple 4

Composition parfumante

On a préparé une composition parfumante de base, destinée à un détergent en poudre, en mélangeant les ingrédients suivants :

| Ingrédients | Parties en poids |
|---|---|
| Acétate de benzyle | 50 |
| Acétate de linalyle | 20 |
| Acétate de phényléthyle | 40 |
| Acétate de verdyle | 100 |
| Aldéhyde undécylénique à 10% * | 70 |
| Aldéhyde hexylcinnamique | 80 |
| Benzoate de méthyle à 10% * | 50 |
| Eugénol | 25 |
| Hélional | 50 |
| 4,4a,5,9b-Tétrahydro-indéno[1,2-d]-1,3-dioxine à 10% * | 50 |
| Iralia® 1) | 60 |
| Iso E Super 2) | 40 |
| Lilial® 3) | 400 |
| Linalol | 100 |
| Méthylacétophénone à 10% * | 20 |
| Méthyl-p-crésol à 10% * | 10 |
| Oxyde de rose à 10% * | 5 |
| Phényléthyl méthyl éther à 10% * | 10 |
| Phénéthylol | 210 |
| Acétate de p-tert-butyl-cyclohexanone | 170 |
| Salicylate de benzyle | 30 |
| Tonalid® 4) | 160 |
| TOTAL | 1750 |

* dans le dipropylèneglycol (DIPG)
1) méthylionone ; origine : Firmenich SA, Genève, Suisse
2) 2-acétyl-1,2,3,4,6,7,8-octahydro-2,3,8,8-tétraméthyl-naphtalène; origine : IFF, USA
3) 2-méthyl-3-(4-tert-butyl-1-phényl)-propanal ; origine : Givaudan-Roure, Vernier, Suisse
4) 7-acétyl-1,1,3,4,4,6-hexaméthyltétraline ; origine : PFW, Hollande

A cette composition de base on a ajouté 250 parties en poids de 5,7-di-tert-butyl-3-méthyl-3H-benzo[b]furan-2-one. On a ainsi obtenu une composition nouvelle dont la note musquée terreuse, aromatique impartie par la Tonalid ® se trouvait nettement renforcée par rapport à celle de la composition de base, l'odeur étant devenue musquée-animale et évoquant clairement le caractère olfactif que l'on aurait pu obtenir si on avait ajouté à la composition de base du Musc Xylol.
D'autre part, lorsque la composition de base contenait, à la place de la Tonalid ® , 60 parties en poids de 3-méthyl-cyclopentadéc-5-én-1-one (voir US 5,354,735), ayant ainsi déjà un caractère musqué, musc-nitré et aussi poudreux, l'addition de 350 parties en poids du composé de l'invention susmentionné a permis d'obtenir une composition nouvelle où l'effet olfactif mentionné plus haut était encore plus évident, l'odeur de la composition évoquant encore plus la fragrance que l'on peut obtenir avec les composés connus tels que le Musc Xylol, le Musc Baur ou encore la Musc cétone.
Par ailleurs, lorsqu'on a ajouté à la composition de base 250 parties en poids de 5,7-diisopropyl-3-méthyl-3H-benzo[b]furan-2-one à la place du composé de l'invention cité plus haut, on a observé des effets olfactifs similaires mais un peu moins marqués.

Exemple 5

Composition parfumante

On a préparé une composition parfumante de base par mélange des ingrédients suivants

| Ingrédients | Parties en poids |
|---|---|
| Essence d'ambre synth. | 100 |

(suite)

| Ingrédients | Parties en poids |
|---|---|
| Essence de bergamote | 100 |
| Exaltex® 1) | 80 |
| Iralia® 2) | 100 |
| Mousse de chêne absolue à 50% * | 270 |
| Mousse cristal | 50 |
| Essence d'orange Portugal Italie | 40 |
| Essence de patchouli | 70 |
| Essence de vétyver Bourbon | 80 |
| Wardia® 3) | 80 |
| TOTAL | 970 |

* dans le dipropylèneglycol (DIPG)

1) oxacyclohexadécan-2-one ; origine : Firmenich SA, Genève, Suisse

2) méthylionone ; origine : Firmenich SA, Genève, Suisse

3) origine : Firmenich SA, Genève, Suisse

Lorsqu'on a ajouté à cette composition de base de type Chypre 30 parties en poids de 3-méthyl-6-phényl-3H-benzo[b]furan-2-one, on a obtenu une composition nouvelle dont l'odeur avait acquis un net caractère fruité-lactonique évoquant des parfums classiques tels que "Madame" de Rochas ou "Champagne" de Yves St. Laurent. D'autre part, lorsqu'on a comparé l'effet imparti par ce composé avec celui que l'on a obtenu en ajoutant à la composition de base 30 parties en poids de γ-undécalactone, on s'est aperçu que la composition obtenue selon l'invention possédait une note fruitée plus marquée, de type abricot, alors que celle de la composition contenant la γ-undécalactone possédait un caractère fruité plus faible et plutôt dans la direction pêche. En plus, après 24 h, la note fruitée était davantage perceptible dans l'odeur de la composition contenant le 3-méthyl-6-phényl-3H-benzo[b]furan-2-one et elle persistait encore une semaine plus tard, sans aucun changement dans la puissance, alors que l'on ne pouvait plus percevoir aucun caractère fruité dans l'odeur de la composition contenant la γ-undécalactone.
L'addition à la composition de base de 30 parties en poids de 6-isopropyl-3-méthyl-3H-benzo[b]furan-2-one a permis d'obtenir une composition nouvelle dont l'odeur possédait un caractère lactonique plus marqué que dans le cas de l'addition de son analogue phénylé, quoique le volume et la ténacité de l'odeur aient été quand même inférieurs.

## Revendications

1. Utilisation à titre d'ingrédient parfumant d'un composé de formule

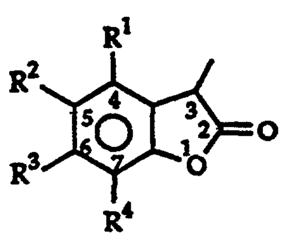

(I)

dans laquelle

a. les symboles $R^1$ à $R^4$ sont identiques ou différents et représentent chacun un atome d'hydrogène, un radical alkyle saturé ou insaturé, linéaire ou ramifié, ayant de 1 à 5 atomes de carbone, un radical alkoxy linéaire ou ramifié, ayant de 1 à 4 atomes de carbone, un radical cycloaliphatique ayant 5 ou 6 atomes de carbone ou un radical aromatique, ce radical cyclique pouvant être substitué par un ou plusieurs radicaux alkyles inférieurs ;
ou dans laquelle

b. deux symboles adjacents parmi les symboles $R^1$ à $R^4$ sont pris ensemble et représentent un cycle, saturé ou insaturé, ayant 5 ou 6 atomes de carbone, ce cycle pouvant être substitué par un ou plusieurs radicaux alkyles inférieurs, et les deux autres symboles représentent l'hydrogène.

**2.** Utilisation selon la revendication 1, d'un composé de formule

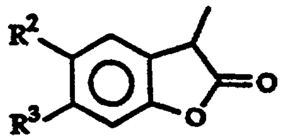

(Ia)

dans laquelle les symboles $R^2$ et $R^3$ sont identiques ou différents et représentent chacun un atome d'hydrogène ou un radical alkyle ou alkoxy de $C_1$ à $C_4$, linéaire ou ramifié, un radical cycloaliphatique ayant 5 ou 6 atomes de carbone ou un radical aromatique, ce radical cyclique pouvant être substitué par un ou plusieurs radicaux alkyles inférieurs.

**3.** Utilisation selon la revendication 1, d'un composé de formule

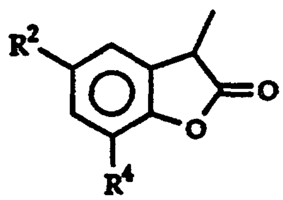

(Ib)

dans laquelle $R^2$ et $R^4$ sont identiques ou différents et représentent chacun un radical alkyle ou alkoxy de $C_1$ à $C_4$, linéaire ou ramifié, un radical cycloaliphatique ayant 5 ou 6 atomes de carbone ou un radical aromatique, ce radical cyclique pouvant être substitué par un ou plusieurs radicaux alkyles inférieurs.

**4.** Utilisation selon la revendication 1 de la 3,4,6-triméthyl-3H-benzo[b]furan-2-one, de la 3,6,7-triméthyl-3H-benzo[b]furan-2-one ou de la 3,5,6,7-tetrahydro-3-méthyl-indéno[5,6-b]furan-2-one.

**5.** Utilisation selon la revendication 2 de l'un des composés suivants:

a. 3-méthyl-3H-benzo[b]furan-2-one;
b. 3,6-diméthyl-3H-benzo[b]furan-2-one;
c. 6-éthyl-3-méthyl-3H-benzo[b]furan-2-one;
d. 6-isopropyl-3-méthyl-3H-benzo[b]furan-2-one;
e. 3-méthyl-6-phényl-3H-benzo[b]furan-2-one;
f. 6-méthoxy-3-méthyl-3H-benzo[b]furan-2-one ;
g. 3-méthyl-5-propyl-3H-benzo[b]furan-2-one;
h. 5-tert-butyl-3-méthyl-3H-benzo[b]furan-2-one ;
i. 3-méthyl-5-(1-méthylpropyl)-3H-benzo[b]furan-2-one ; et
j. 3,5,6-triméthyl-3H-benzo[b]furan-2-one.

**6.** Utilisation selon la revendication 3 de la 5,7-diisopropyl-3-méthyl-3H-benzo[b]furan-2-one ou de la 5,7-di-tert-butyl-3-méthyl-3H-benzo[b]furan-2-one.

**7.** Utilisation selon la revendication 6, de la 5,7-di-tert-butyl-3-méthyl-3H-benzo[b]furan-2-one en mélange avec la 3-méthyl-cyclopentadéc-5-én-1-one.

**8.** Composition parfumante ou article parfumé contenant à titre d'ingrédient actif un composé de formule (I), (Ia) ou (Ib) telle que définie à la revendication 1, 2, respectivement 3.

**9.** Composition parfumante ou article parfumé selon la revendication 8, contenant l'un des composés dtés aux revendications 4 à 6.

**10.** Composition parfumante ou article parfumé selon la revendication 9, contenant la 5,7-di-tert-butyl-3-méthyl-3H-benzo[b]furan-2-one en mélange avec la 3-méthyl-cyclopentadéc-5-én-1-one.

**11.** Article parfumé selon l'une des revendications 8 à 10, sous forme d'un parfum ou d'une eau de toilette, d'un savon, d'un gel de bain ou douche, d'un shampoing ou autre produit d'hygiène capillaire, d'une préparation cosmétique, d'un désodorisant corporel ou d'air ambiant, d'un détergent, d'un adoucissant textile ou d'un produit d'entretien.

**12.** Composé de formule

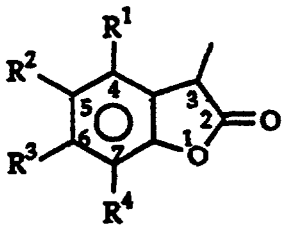

(I)

telle que définie à la revendication 1, alinéa a., étant entendu que les combinaisons suivantes sont exclues :

a. $R^1 = R^2 = R^3 = R^4 = H$;
b. $R^1 = R^2 = R^4 = H$ et $R^3$ = méthyle ou méthoxy ou phényle ou 2-méthylpropyle ou cyclopentyle ou cyclohexyle ;
c. $R^1 = R^3 = R^4 = H$ et $R^2$ = méthyle ou éthyle ou phényle ;
d. $R^1 = R^4 = H$, $R^2 = CH_3$ et $R^3$ = cyclopentyle ou cydlohexyle;
e. $R^1 = R^4 = H$, $R^2$ = phényle et $R^3$ = méthoxy;
f. $R^1 = R^3$ = méthoxy et, soit $R^2 = CH_3$ et $R^4 = H$, soit $R^2 = H$ et $R^4 = CH_3$; et
g. $R^1 = R^4 = H$, $R^2$ = méthoxy et $R^3$ = cyclohexyle;

ou telle que définie à la revendication 1, alinéa b., mais en excluant la 1,5,8-triméthyl-1H-naphto[2,1-b]furan-2-one, la 3-méthyl-3H-naphto[2,3-b]furan-2-one et la 1-méthyl-1H-naphto[2,1-b]furan-2-one.

**13.** Composé de formule

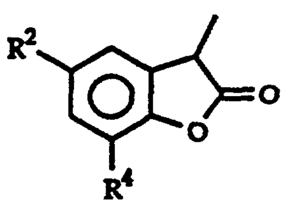

(Ib)

dans laquelle $R^2$ et $R^4$ sont identiques ou différents et représentent chacun un radical alkyle ou alkoxy de $C_1$ à $C_4$, linéaire ou ramifié ou un radical cyclique saturé ou insaturé, ayant 5 ou 6 atomes de carbone et pouvant être substitué par un ou plusieurs radicaux alkyles inférieurs.

**14.** Composé selon la revendication 12 ou 13, choisi dans le groupe constitué par :

a. 6-éthyl-3-méthyl-3H-benzo[b]furan-2-one ;
b. 6-isopropyl-3-méthyl-3H-benzo[b]furan-2-one;
c. 3-méthyl-5-propyl-3H-benzo[b]furan-2-one;
d. 5-tert-butyl-3-méthyl-3H-benzo[b]furan-2-one;
e. 3-méthyl-5-(1-méthylpropyl)-3H-benzo[b]furan-2-one;
f. 3,4,6-triméthyl-3H-benzo[b]furan-2-one;
g. 3,5,6-triméthyl-3H-benzo[b]furan-2-one;
h. 3,6,7-triméthyl-3H-benzo[b]furan-2-one;

i. 3,5,6,7-tétrahydro-3-méthyl-indéno[5,6-b]furan-2-one;
j. 5,7-diisopropyl-3-méthyl-3H-benzo[b]furan-2-one; et
k. 5,7-di-tert-butyl-3-méthyl-3H-benzo[b]furan-2-one.

**15.** Procédé pour la préparation d'un composé de formule (I) telle que définie à la revendication 12, **caractérisé en ce qu'**on fait réagir un dérivé phénolique de formule

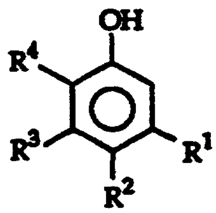

(II)

dans laquelle les symboles $R^1$ à $R^4$ sont définis comme à la formule (I), avec le méthylglyoxal, en présence d'un catalyseur acide et dans un solvant organique susceptible de former un mélange azéotropique avec l'eau.

**16.** Procédé selon la revendication 15, **caractérisé en ce que** le catalyseur acide est l'acide p-toluènesulfonique et le solvant organique est le toluène.

## Patentansprüche

**1.** Verwendung als Riechstoff einer Verbindung der Formel

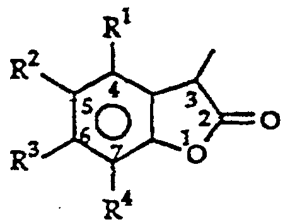

(I),

worin

a. die Symbole $R^1$ bis $R^4$ identisch oder voneinander verschieden sind und jeweils für ein Wasserstoffatom, einen gesättigten oder ungesättigten, geradkettigen oder verzweigten Alkylrest mit 1 bis 5 Kohlenstoffatomen, einen geradkettigen oder verzweigten Alkoxyrest mit 1 bis 4 Kohlenstoffatomen, einen cycloaliphatischen Rest mit 5 oder 6 Kohlenstoffatomen oder einen aromatischen Rest stehen, wobei dieser cyclische Rest mit einem oder mehreren niederen Alkylresten substituiert sein kann,
oder worin
b. zwei nebeneinander gelegene Symbole unter den Symbolen $R^1$ bis $R^4$ zusammengenommen für einen gesättigten oder ungesättigten Ring mit 5 oder 6 Kohlenstoffatomen stehen, wobei dieser Ring mit einem oder mehreren niederen Alkylresten substituiert sein kann, und die beiden weiteren Symbole für Wasserstoff stehen.

**2.** Verwendung nach Anspruch 1 einer Verbindung der Formel

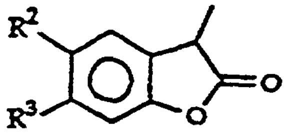

(Ia),

worin die Symbole $R^2$ und $R^3$ identisch oder voneinander verschieden sind und jeweils für ein Wasserstoffatom oder einen geradkettigen oder verzweigten $C_1$-$C_4$-Alkyl- oder Alkoxyrest, einen cycloaliphatischen Rest mit 5 oder 6 Kohlenstoffatomen oder einen aromatischen Rest stehen, wobei dieser cyclische Rest mit einem oder mehreren niederen Alkylresten substituiert sein kann.

**3.** Verwendung nach Anspruch 1 einer Verbindung der Formel

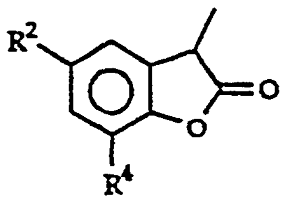

(Ib),

worin $R^2$ und $R^4$ identisch oder voneinander verschieden sind und jeweils für einen geradkettigen oder verzweigten $C_1$-$C_4$-Alkyl- oder Alkoxyrest, einen cycloaliphatischen Rest mit 5 oder 6 Kohlenstoffatomen oder einen aromatischen Rest stehen, wobei dieser cyclische Rest mit einem oder mehreren niederen Alkylresten substituiert sein kann.

**4.** Verwendung nach Anspruch 1 von 3,4,6-Trimethyl-3H-benzo[b]furan-2-on, 3,6,7-Trimethyl-3H-benzo[b]furan-2-on oder 3,5,6,7-Tetrahydro-3-methyl-indeno[5,6-b]furan-2-on.

**5.** Verwendung nach Anspruch 2 von einer der folgenden Verbindungen:

a. 3-Methyl-3H-benzo[b]furan-2-on;
b. 3,6-Dimethyl-3H-benzo[b]furan-2-on;
c. 6-Ethyl-3-methyl-3H-benzo[b]furan-2-on;
d. 6-Isopropyl-3-methyl-3H-benzo[b]furan-2-on;
e. 3-Methyl-6-phenyl-3H-benzo[b]furan-2-on;
f. 6-Methoxy-3-methyl-3H-benzo[b]furan-2-on;
g. 3-Methyl-5-propyl-3H-benzo[b]furan-2-on;
h. 5-*tert*.-Butyl-3-methyl-3H-benzo[b]furan-2-on;
i. 3-Methyl-5-(1-methylpropyl)-3H-benzo[b]furan-2-on; und
j. 3,5,6-Trimethyl-3H-benzo[b]furan-2-on.

**6.** Verwendung nach Anspruch 3 von 5,7-Diisopropyl-3-methyl-3H-benzo[b]furan-2-on oder 5,7-Di-*tert*.-butyl-3-methyl-3H-benzo[b]furan-2-on.

**7.** Verwendung nach Anspruch 6 von 5,7-Di-*tert*.-butyl-3-methyl-3H-benzo[b]furan-2-on in Mischung mit 3-Methylcyclopentadec-5-en-1-on.

**8.** Duftzusammensetzung oder parfümierter Artikel, welche bzw. welcher als Riechstoff eine Verbindung der Formel (I), (Ia) oder (Ib) gemäß der Definition in Anspruch 1, 2 bzw. 3 enthält.

**9.** Duftzusammensetzung oder parfümierter Artikel nach Anspruch 8, welche bzw. welcher eine der in den Ansprüchen 4 bis 6 genannten Verbindungen enthält.

**10.** Duftzusammensetzung oder parfümierter Artikel nach Anspruch 9, welche bzw. welcher 5,7-Di-*tert*.-butyl-3-methyl-3H-benzo[b]furan-2-on in Mischung mit 3-Methyl-cyclopentadec-5-en-1-on enthält.

**11.** Parfümierter Artikel nach einem der Ansprüche 8 bis 10 in Form eines Parfüms oder eines Eau de Toilette, einer Seife, eines Dusch- oder Badegels, eines Shampoo oder eines anderen Haarpflegeproduktes, eines kosmetischen Präparats, eines Körper- oder Raumluftdeodorants, eines Detergens, eines Textilweichspülers oder eines Allzweckreinigers.

**12.** Verbindung der Formel

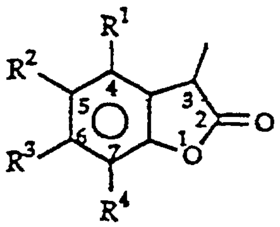

(I)

gemäß der Definition von Anspruch 1, Punkt a., unter der Maßgabe, daß die folgenden Kombinationen ausgeschlossen sind:

a. $R^1 = R^2 = R^3 = R^4 = H$;
b. $R^1 = R^2 = R^4 = H$ und $R^3$ = Methyl oder Methoxy oder Phenyl oder 2-Methylpropyl oder Cyclopentyl oder Cyclohexyl;
c. $R^1 = R^3 = R^4 = H$ und $R^2$ = Methyl oder Ethyl oder Phenyl;
d. $R^1 = R^4 = H$, $R^2 = CH_3$ und $R^3$ = Cyclopentyl oder Cyclohexyl;
e. $R^1 = R^4 = H$, $R^2$ = Phenyl und $R^3$ = Methoxy;
f. $R^1 = R^3$ = Methoxy und entweder $R^2 = CH_3$ und $R^4 = H$ oder $R^2 = H$ und $R^4 = CH_3$; und
g. $R^1 = R^4 = H$, $R^2$ = Methoxy und $R^3$ = Cyclohexyl;

oder gemäß der Definition von Anspruch 1, Punkt b., jedoch unter Ausschluß von 1,5,8-Trimethyl-1H-naphtho[2,1-b]furan-2-on, 3-Methyl-3H-naphtho[2,3-b]furan-2-on und 1-Methyl-1H-naphtho[2,1-b]furan-2-on.

**13.** Verbindung der Formel

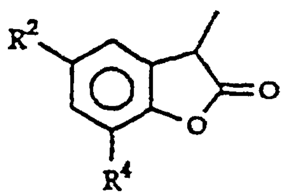

(Ib),

worin $R^2$ und $R^4$ identisch oder voneinander verschieden sind und jeweils für einen geradkettigen oder verzweigten $C_1$-$C_4$-Alkyl- oder Alkoxyrest oder einen gesättigten oder ungesättigten cyclischen Rest mit 5 oder 6 Kohlenstoffatomen stehen und mit einem oder mehreren niederen Alkylresten substituiert sein können.

**14.** Verbindung nach Anspruch 12 oder 13, ausgewählt aus der Gruppe bestehend aus:

a. 6-Ethyl-3-methyl-3H-benzo[b]furan-2-on;
b. 6-Isopropyl-3-methyl-3H-benzo[b]furan-2-on;
c. 3-Methyl-5-propyl-3H-benzo[b]furan-2-on;
d. 5-tert.-Butyl-3-methyl-3H-benzo[b]furan-2-on;
e. 3-Methyl-5-(1-methylpropyl)-3H-benzo[b]furan-2-on;
f. 3,4,6-Trimethyl-3H-benzo[b]furan-2-on;
g. 3,5,6-Trimethyl-3H-benzo[b]furan-2-on;

h. 3,6,7-Trimethyl-3H-benzo[b]furan-2-on;
i. 3,5,6,7-Tetrahydro-3-methyl-indeno[5,6-b]furan-2-on;
j. 5,7-Diisopropyl-3-methyl-3H-benzo[b]furan-2-on; und
k. 5,7-Di-*tert.*-butyl-3-methyl-3H-benzo[b]furan-2-on.

**15.** Verfahren zur Herstellung einer Verbindung der Formel (I) gemäß der Definition in Anspruch 12, **dadurch gekennzeichnet, daß** man ein Phenolderivat der Formel

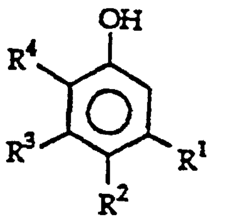

(II),

worin die Symbole $R^1$ bis $R^4$ wie in Formel (I) definiert sind, mit Methylglyoxal in Gegenwart eines sauren Katalysators und in einem organischen Lösungsmittel reagieren läßt, welches in der Lage ist, mit Wasser eine azeotrope Mischung zu bilden.

**16.** Verfahren nach Anspruch 15, **dadurch gekennzeichnet, daß** der saure Katalysator *p*-Toluolsulfonsäure und das organische Lösungsmittel Toluol ist.

## Claims

**1.** Use as a perfuming ingredient of a compound of formula

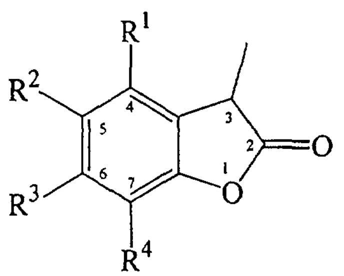

(I)

wherein

a. symbols $R^1$ to $R^4$ are identical or different and represent each a hydrogen atom, a saturated or unsaturated, linear or branched, alkyl radical having from 1 to 5 carbons, a linear or branched alkoxy group having from 1 to 4 carbons, a cycloaliphatic radical having 5 or 6 carbon atoms or an aromatic radical, which cyclic radical can possess one or several lower alkyl radicals as substituents ; or
b. two adjacent symbols amongst $R^1$ to $R^4$ symbols are taken together and represent a saturated or unsaturated ring having 5 or 6 carbon atoms, which ring can possess one or several lower alkyl radicals as substituents, and the other two symbols represent hydrogen.

**2.** Use according to claim 1, of a compound of formula

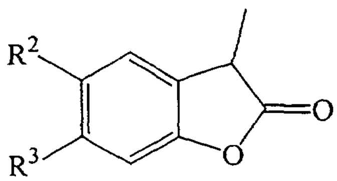

(Ia)

wherein $R^2$ and $R^3$ are identical or different and each represents a hydrogen atom or a $C_1$ to $C_4$ linear or branched alkyl or alkoxy radical, a cycloaliphatic radical having 5 or 6 carbon atoms or an aromatic radical, and wherein this cyclic radical can possess one or several lower alkyl radical as a substituent.

**3.** Use according to claim 1, of a compound of formula

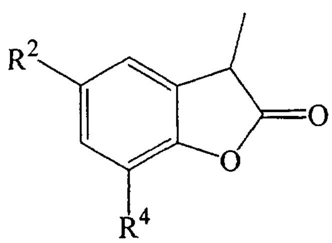

(Ib)

wherein $R^2$ and $R^4$ are identical or different and each represents a $C_1$ to $C_4$ linear or branched alkyl or alkoxy radical, a cycloaliphatic radical having 5 or 6 carbon atoms or an aromatic radical, and wherein this cyclic radical can possess one or several lower alkyl radical as a substituent.

**4.** Use according to claim 1, of 3,4,6-trimethyl-3H-benzo[b]furan-2-one, 3,6,7-trimethyl-3H-benzo[b]furan-2-one or 3,5,6,7-tetrahydro-3-methyl-indeno[5,6-b]furan-2-one.

**5.** Use according to claim 2, of one of the following compounds :

a. 3-methyl-3H-benzo[b]furan-2-one ;
b. 3,6-dimethyl-3H-benzo[b]furan-2-one;
c. 6-ethyl-3-methyl-3H-benzo[b]furan-2-one ;
d. 6-isopropyl-3-methyl-3H-benzo[b]furan-2-one ;
e. 3-methyl-6-phenyl-3H-benzo[b]furan-2-one;
f. 6-methoxy-3-methyl-3H-benzo[b]furan-2-one ;
g. 3-methyl-5-propyl-3H-benzo[b]furan-2-one;
h. 5-tert-butyl-3-methyl-3H-benzo[b]furan-2-one ;
i. 3-methyl-5-(1-methylpropyl)-3H-benzo[b]furan-2-one ; and
j. 3,5,6-trimethyl-3H-benzo[b]furan-2-one.

**6.** Use according to claim 3, of 5,7-diisopropyl-3-methyl-3H-benzo[b]furan-2-one or of 5,7-di-tert-butyl-3-methyl-3H-benzo[b]furan-2-one.

**7.** Use according to claim 6, of 5,7-di-tert-butyl-3-methyl-3H-benzo[b]furan-2-one in admixture with 3-methyl-cyclopentadec-5-en-1-one.

**8.** Perfuming composition or perfumed article containing as an active perfuming ingredient a compound of formula (I), (Ia) or (Ib) as defined in claim 1, 2, respectively 3.

**9.** Perfuming composition or perfumed article according to claim 8, containing one of the compounds recited in claims 4 to 6.

**10.** Perfuming composition or perfumed article according to claim 9, containing 5,7-di-tert-butyl-3-methyl-3H-benzo[b]furan-2-one in admixture with 3-methyl-cyclopentadec-5-en-1-one.

**11.** Perfumed article according to anyone of claims 8 to 10, in the form of a perfume or cologne, a soap, a bath or shower gel, a shampoo or other haircare product, a cosmetic preparation, a body or air deodorant, a detergent, a fabric softener or a household product.

**12.** A compound of formula

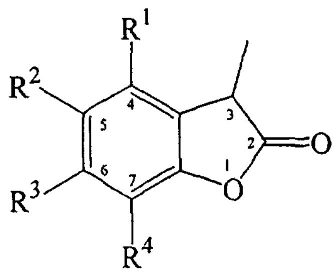

(I)

as defined in claim 1, paragraph a., provided that the following combinations are excluded:

a. $R^1 = R^2 = R^3 = R^4 = H$ ;
b. $R^1 = R^2 = R^4 = H$ and $R^3$ = methyl or methoxy or phenyl or 2-methylpropyl or cyclopentyl or cyclohexyl;
c. $R^1 = R^3 = R^4 = H$ and $R^2$ = methyl or ethyl or phenyl;
d. $R^1 = R^4 = H$, $R^2 = CH_3$ and $R^3$ = cyclopentyl or cyclohexyl;
e. $R^1 = R^4 = H$, $R^2$ = phenyl and $R^3$ = methoxy ;
f. $R^1 = R^3$ = methoxy and, either $R^2 = CH_3$ and $R^4 = H$, or $R^2 = H$ and $R^4 = CH_3$ ; and
g. $R^1 = R^4 = H$, $R^2$ = methoxy and $R^3$ = cyclohexyl;

or as defined in claim 1, paragraph b., provided that 1,5,8-trimethyl-lH-naphtho[2,1-b]furan-2-one, 3-methyl-3H-naphtho[2,3-b]furan-2-one and 1-methyl-1H-naphtho[2,1-b]furan-2-one are excluded.

**13.** A compound of formula

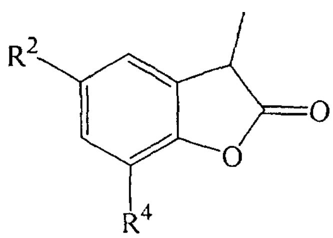

(Ib)

in which $R^2$ and $R^4$ are identical or different and each represents a $C_1$ to $C_4$ alkyl or alkoxy radical, linear or branched, or a saturated or unsaturated cyclic radical having 5 or 6 carbon atoms, and wherein said cyclic radical can possess one or several lower alkyl radicals as substituents.

**14.** A compound according to claim 12 or 13, selected from the group consisting of:

a. 6-ethyl-3-methyl-3H-benzo[b]furan-2-one ;
b. 6-isopropyl-3-methyl-3H-benzo[b]furan-2-one;
c. 3-methyl-5-propyl-3H-benzo[b]furan-2-one ;
d. 5-tert-butyl-3-methyl-3H-benzo[b]furan-2-one ;
e. 3-methyl-5-(1-methylpropyl)-3H-benzo[b]furan-2-one ;
f. 3,4,6-trimethyl-3H-benzo[b]furan-2-one ;
g. 3,5,6-trimethyl-3H-benzo[b]furan-2-one ;
h. 3,6,7-trimethyl-3H-benzo[b]furan-2-one ;
i. 3,5,6,7-tetrahydro-3-methyl-indeno[5,6-b]furan-2-one;
j. 5,7-diisopropyl-3-methyl-3H-benzo[b]furan-2-one ; and
k. 5,7-di-tert-butyl-3-methyl-3H-benzo[b]furan-2-one.

**15.** A process for the preparation of a compound of formula (I) as defined in claim 12, **characterized in that** a phenolic derivative of formula

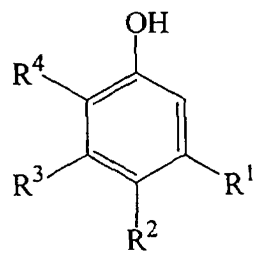

(II)

in which the symbols $R^1$ to $R^4$ are defined as for formula (I), is reacted with methylglyoxal, in the presence of an acidic catalyst and in an organic solvent susceptible of forming an azeotropic mixture with water.

**16.** A process according to claim 15, **characterized in that** the acidic catalyst is p-toluenesulphonic acid and the organic solvent is toluene.